# EUROPEAN PATENT APPLICATION

(11) **EP 3 578 204 A1**
(43) Date of publication of application: **11.12.2019**
(21) Application number: 18748653.5
(22) Date of filing: 02.02.2018
(51) Int. Cl.: A61K 47/60, A61K 47/68, A61K 38/28

(54) **CONJUGATE OF BIOACTIVE MATERIAL HAVING ENHANCED SUSTAINABILITY AND USE THEREOF**

(30) Priority: 03.02.2017 KR 20170015663; 29.09.2017 KR 20170127187
(71) Applicant: Hanmi Pharm. Co., Ltd., Hwaseong-si, Gyeonggi-do 18536 (KR)
(72) Inventor: PARK, Young Jin, Hwaseong-si Gyeonggi-do 18469 (KR); LEE, Jong-Soo, Hwaseong-si Gyeonggi-do 18469 (KR); CHOI, In Young, Hwaseong-si Gyeonggi-do 18469 (KR)
(74) Representative: Mewburn Ellis LLP
(86) International application number: PCT/KR2018/001458
(87) International publication number: WO 2018/143729

(57) **Abstract**

The present invention relates to a conjugate including a physiologically active material, a linker, and a material capable of increasing *in vivo* half-life of the physiologically active material, a method for preparing the same, and a preparation thereof.

## Description

### [Technical Field]

The present invention relates to a conjugate which includes a physiologically active material, a linker, and a material capable of increasing *in vivo* half-life of the physiologically active material; a method for preparing the same; and a preparation thereof.

### [Background Art]

In general, physiologically active polypeptides are easily denatured and degraded by *in vivo* proteases due to their poor stability or are relatively easily removed by the kidneys due to their relatively small size. Accordingly, to maintain their blood concentration and titer, it is necessary that protein drugs containing these physiologically active polypeptides as pharmaceutical ingredients be more frequently administered to patients. However, in the case of protein drugs mostly administered to patients in the form of injections, frequent administration via injections is required to maintain the blood concentration of the physiologically active polypeptides, which cause pain to the patients and also incur high treatment costs. To solve these problems, many efforts have been made to maximize the efficacy of protein drugs by increasing their blood stability while maintaining their blood concentration at a high level for a long period of time.

Meanwhile, insulin is a blood glucose level-controlling hormone secreted by the pancreas of the human body and serves to maintain blood glucose levels within a normal range while transporting excess glucose in the blood to cells, thereby supplying an energy source to the cells. However, diabetic patients cannot maintain normal insulin functions due to insulin deficiency, insulin resistance, or loss of beta cell function, and thus the glucose in the blood cannot be used as an energy source. As a result, diabetic patients show a symptom of high blood glucose levels called hyperglycemia and eventually excrete glucose into the urine, which is associated with several complications.

Accordingly, insulin therapy is essential for diabetic patients with inability to produce insulin (type I) or insulin resistance (type II), and the blood glucose levels of these diabetic patients can be kept at normal levels by insulin administration. However, like other proteins and peptide hormones, insulin has an extremely short *in vivo* half-life and thus has a disadvantage in that it must be repeatedly administered, and frequent administration leads to severe pain and inconvenience to the patients. Accordingly, studies have been conducted to develop various types of protein formulations, chemical conjugates (fatty acid conjugates and polyethylene polymer conjugates), *etc.* so as to improve the quality of life of the patients by reducing the frequency of administration by increasing *in vivo* half-life of these proteins. Examples of long-acting insulin preparations currently on the market include insulin glargine, Lantus® (Sanofi-Aventis: about 20 to 22 hours of duration); insulin detemir, Levemir® (Novo Nordisk: about 18 to 22 hours of duration); and insulin degludec, Tresiba® (about 40 hours of duration). These long-acting insulin preparations are suitable as basal insulin because there is no peak of insulin concentration in the blood, but they still have the inconvenience of requiring administration once or twice daily because their half-life is not long enough. Accordingly, there is a limit to achieving the object of increasing patient convenience by significantly lowering the frequency of administration in diabetic patients requiring long-term administration.

Several previous papers, such as Authier F et al. (Biochem J. 1998 Jun 1; 332 (Pt 2): 421 to 30), Duckworth WC et al. (Endocr Rev. 1998 Oct; 19(5): 608 to 24.), and Valera Mora ME et al. (J Am Coll Nutr. 2003 Dec; 22(6): 487 to 93), describe the process of *in vivo* removal of insulin. Reviewing these papers, it is known that more than 50% of insulin is removed from the kidneys and the remaining insulin is eliminated through receptor-mediated clearance (RMC) at the target sites such as muscle, fat, liver, *etc.*

Accordingly, there is a growing need for the development of physiologically active material preparations, in particular insulin preparations, which can reduce the frequency of administration in patients because of significantly increased blood half-life while being capable of avoiding the RMC.

### [Disclosure]

### [Technical Problem]

An object of the present invention is to provide a conjugate of a physiologically active material for the purpose of extending *in vivo* half-life of the physiologically active material.

Specifically, the present invention intends to provide a conjugate of a physiologically active material, in which a physiologically active material and a material capable of increasing *in vivo* half-life of the physiologically active material are linked through polyethylene glycol, and the polyethylene glycol has a size of greater than 0 kDa to less than 3.4 kDa.

Another object of the present invention is to provide a composition containing the conjugate.

Specifically, the present invention intends to provide a long-acting preparation with improved *in vivo* duration and stability containing the conjugate.

Additionally, the present invention intends to provide a preparation for preventing or treating diabetes containing the conjugate.

Still another object of the present invention is to provide a method for preparing the conjugate.

Still another object of the present invention is to provide a use of the conjugate or composition containing the conjugate for use in the preparation of a medicament.

### [Technical Solution]

To achieve the above objects, an aspect of the present invention provides a conjugate of a physiologically active material for the purpose of extending *in vivo* half-life of the physiologically active material.

In a specific embodiment, the present invention relates to a conjugate of a physiologically active material in which a physiologically active material and a material capable of increasing *in vivo* half-life of the physiologically active material are linked through polyethylene glycol, and the polyethylene glycol has a size of greater than 0 kDa to less than 3.4 kDa.

In a conjugate according to a previous specific embodiment, the conjugate is represented by Formula 1 below:

[Formula 1] X-L-F

in which:
X is a physiologically active material;
L, being a linker, is polyethylene glycol having a size of greater than 0 kDa to less than 3.4 kDa; and
F is a material capable increasing *in vivo* half-life of the physiologically active material.

In a conjugate according to any one of the previous specific embodiments, the conjugate exhibits an increased *in vivo* half-life compared to a conjugate which only differs from the conjugate in that L is polyethylene glycol having a size of 3.4 kDa.

In a conjugate according to any one of the previous specific embodiments, L is polyethylene glycol having a size of greater than 0 kDa to 3 kDa or less.

In a conjugate according to any one of the previous specific embodiments, the physiologically active material is a physiologically active polypeptide.

In a conjugate according to any one of the previous specific embodiments, the physiologically active material selected from the group consisting of toxins; glucagon-like peptide-1 (GLP-1) receptor agonists; glucagon receptor agonists; gastric inhibitory polypeptide (GIP) receptor agonists; fibroblast growth factor (FGF) receptor agonists; cholecystokinin receptor agonists; gastrin receptor agonists; melanocortin receptor agonists; materials binding to two or more receptors among GLP receptor, glucagon receptor, and GIP receptor; somatostatin; peptide YY (PYY); neuropeptide Y (NPY); oxyntomodulin; fibroblast growth factor (FGF); bradykinin; eledoisin; oxytocin; vasopressin; sermorelin; prolactin-releasing peptides; orexin; thyroid-releasing peptides; calmodulin; motilin; vasoactive intestinal peptides; atrial natriuretic peptides (ANP); C-type natriuretic peptides (CNP); neurokinin A; neuromedin; renin; endothelin; sarafotoxin peptides; carsomorphin peptides; dermorphin; dynorphin; endorphin; enkepalin; tumor necrosis factor receptors; urokinase receptors; thymopoietin; thymulin; thymopentin; tymosin; thymic humoral factors; adrenomodullin; allatostatin; amyloid β-protein fragments; antibiotic peptides; antioxidant peptides; bombesin; osteocalcin; CART peptides; E-selectin; intercellular adhesion molecule 1 (ICAM-1); vascular cell adhesion molecule 1 (VCAM-1); leucokine; kringle-5; laminin; inhibin; galanin; fibronectin; pancreastatin; fuzeon; glucagon-like peptides (GLP-1 or GLP-2, etc); G protein-coupled receptors; erythropoietic growth factors; leukopoietin; amylin; human growth hormone; growth hormone-releasing hormone; growth hormone-releasing peptides; interferons; interferon receptors; colony-stimulating factors; interleukins; interleukin receptors; enzymes; interleukin-binding proteins; cytokine-binding proteins; macrophage-activating factors; macrophage peptides; B cell factors; T cell factors; protein A; allergy-inhibiting factors; necrosis glycoproteins; immunotoxins; lymphotoxins; tumor necrosis factors; tumor suppressors; transforming growth factors; α-1 antitrypsin; albumin; α-lactalbumin; apolipoprotein-E; erythropoietin; high-glycosylated erythropoietin; angiopoietins; hemoglobins; thrombin; thrombin receptor-activating peptides; thrombomodulin; blood factor VII; blood factor VIIa; blood factor VIII; blood factor IX; blood factor XIII; plasminogen activators; fibrin-binding peptides; urokinase; streptokinase; hirudin; protein C; C-reactive protein; renin inhibitors; collagenase inhibitors; superoxide dismutase; leptin; platelet-derived growth factor; epithelial growth factor; epidermal growth factor; angiostatin; angiotensin; bone morphogenetic growth factor; bone morphogenetic protein; calcitonin; insulin; atriopeptin; cartilage-inducing factor; elcatonin; connective tissue-activating factor; tissue factor pathway inhibitor; follicle-stimulating hormone; luteinizing hormone; luteinizing hormone-releasing hormone; nerve growth factors; axogenesis factor-1; brain-natriuretic peptide; glial-derived neurotrophic factor; netrin; neutrophil inhibitory factor; neurotrophic factor; neurturin; parathyroid hormone; relaxin; secretin; somatomedin; insulin-like growth factor; adrenocortical hormone; glucagon; cholecystokinin; pancreatic polypeptides; gastrin-releasing peptides; gastrin inhibitory peptides; corticotropin-releasing factor; thyroid-stimulating hormone; autotaxin; lactoferrin; myostatin; activity-dependent neuroprotective protein (ADNP), β-secretase1 (BACE1), amyloid precursor protein (APP), neural cell adhesion molecule (NCAM), amyloid β, tau, receptor for advanced glycation endproducts (RAGE), α-synuclein, or agonists or antagonists thereof; receptors, receptor agonists; cell surface antigens; monoclonal antibody; polyclonal antibody; antibody fragments; virus-derived vaccine antigens; hybrid polypeptides or chimeric polypeptides that activate at least one receptor agonist; and analogues thereof.

In a conjugate according to any one of the previous specific embodiments, the physiologically active material simultaneously activates at least two receptors.

In a conjugate according to any one of the previous specific embodiments,
the toxin is selected from the group consisting of maytansine or a derivative thereof, auristatin or a derivative thereof, duocarmycin or a derivative thereof, and pyrrolobenzodiazepine (PBD) or a derivative thereof;
the glucagon-like peptide-1 (GLP-1) receptor agonist is selected from the group consisting of native glucagon-like peptide-1 (GLP-1), native exendin-3, native exendin-4, and analogues thereof;
the FGF receptor agonist is selected from the group consisting of FGF1 or an analogue thereof, FGF19 or an analogue thereof, FGF21 or an analogue thereof, and FGF23 or an analogue thereof;
the interferon is selected from the group consisting of interferon-a, interferon-β, and interferon-γ;
the interferon receptor is selected from the group consisting of interferon-a receptor, interferon-β receptor, interferon-y receptor, and soluble type I interferon receptors;
the interleukin is selected from the group consisting of interleukin-1, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18, interleukin-19, interleukin-20, interleukin-21, interleukin-22, interleukin-23, interleukin-24, interleukin-25, interleukin-26, interleukin-27, interleukin-28, interleukin-29, and interleukin-30;
the interleukin receptor is interleukin-1 receptor or interleukin-4 receptor;
the enzyme is selected from the group consisting of β-glucosidase, α-galactosidase, β-galactosidase, iduronidase, iduronate-2-sulfatase, galactose-6-sulfatase, acid α-glucosidase, acid ceramidase, acid sphingomyelinase, galactocerebrosidase, arylsulfatase A, arylsulfatase B, β-hexosaminidase A, β-hexosaminidase B, heparin *N*-sulfatase, α-D-mannosidase, β-glucuronidase, N-acetylgalactosamine-6 sulfatase, lysosomal acid lipase, α-*N*-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, myeloperoxidase, α-galactosidase-A, agalsidase α, agalsidase β, α-L-iduronidase, butyrylcholinesterase, chitinase, glutamate decarboxylase, and imiglucerase;
the interleukin-binding protein is IL-18bp;
the cytokine-binding protein is tumor necrosis factor (TNF)-binding protein;
the nerve growth factors are selected from the group consisting of nerve growth factor, ciliary neurotrophic factor, axogenesis factor-1, brain-natriuretic peptide, glial-derived neurotrophic factor, netrin, neutrophil inhibitory factor, neurotrophic factor, and neurturin;
the myostatin receptor is selected from the group consisting of TNFR (P75), TNFR (P55), IL-1 receptor, VEGF receptor, and B cell activating factor receptor;
the myostatin receptor antagonist is IL1-Ra;
the cell surface antigen is selected from the group consisting of CD2, CD3, CD4, CD5, CD7, CD11a, CD11b, CD18, CD19, CD20, CD23, CD25, CD33, CD38, CD40, CD45, and CD69; and
the antibody fragments are selected from the group consisting of scFv, Fab, Fab', F(ab')₂, and Fd.

In a conjugate according to any one of the previous specific embodiments, the physiologically active material is native exendin-3 or an analogue thereof; native exendin-4 or an analogue thereof; native insulin or an analogue thereof; native GLP-1 or an analogue thereof; native GLP-2 or an analogue thereof; native oxyntomodulin or an analogue thereof; native glucagon or an analogue thereof; native fibroblast growth factor or an analogue thereof; native ghrelin or an analogue thereof; native calcitonin or an analogue thereof; native granulocyte-colony stimulating factor or an analogue thereof; or a material binding to two or more receptors among a GLP receptor, a glucagon receptor, and a GIP receptor.

In a conjugate according to any one of the previous specific embodiments, the physiologically active material is native insulin or an insulin analogue which has a reduced binding affinity for an insulin receptor compared to native insulin.

In a conjugate according to any one of the previous specific embodiments, the insulin analogue is in a two-chain form including both the A chain and the B chain.

In a conjugate according to any one of the previous specific embodiments, the insulin analogue has a reduced binding affinity for an insulin receptor compared to native insulin and includes at least one amino acid modification or deletion in the A chain or B chain of native insulin.

In a conjugate according to any one of the previous specific embodiments, the insulin analogue is an insulin analogue in which at least one amino acid, selected from the group consisting of the 1^{st} amino acid, the 2^{nd} amino acid, the 3^{rd} amino acid, the 5^{th} amino acid, the 8^{th} amino acid, the 10^{th} amino acid, the 12^{th} amino acid, the 16^{th} amino acid, the 23^{rd} amino acid, the 24^{th} amino acid, the 25^{th} amino acid, the 26^{th} amino acid, the 27^{th} amino acid, the 28^{th} amino acid, the 29^{th} amino acid, and the 30^{th} amino acid of the insulin B chain, and the 1^{st} amino acid, the 2^{nd} amino acid, the 5^{th} amino acid, the 8^{th} amino acid, the 10^{th} amino acid, the 12^{th} amino acid, the 14^{th} amino acid, the 16^{th} amino acid, the 17^{th} amino acid, the 18^{th} amino acid, the 19^{th} amino acid, and the 21^{st} amino acid of the insulin A chain, is substituted with a different amino acid or deleted.

In a conjugate according to any one of the previous specific embodiments, the insulin analogue is an insulin analogue in which at least one amino acid, selected from the group consisting of the 8^{th} amino acid, the 23^{rd} amino acid, the 24^{th} amino acid, and the 25^{th} amino acid of the native insulin B chain, and the 1^{st} amino acid, the 2^{nd} amino acid, the 14^{th} amino acid, and the 19^{th} amino acid of the native insulin A chain, is substituted with a different amino acid.

In a conjugate according to any one of the previous specific embodiments, the substituting different amino acid is selected from the group consisting of alanine, glutamic acid, asparagine, isoleucine, valine, glutamine, glycine, lysine, histidine, cysteine, phenylalanine, tryptophan, proline, serine, threonine, and aspartic acid.

In a conjugate according to any one of the previous specific embodiments, the insulin analogue has a reduced binding affinity for an insulin receptor due to a deletion in at least one amino acid of the A chain or B chain of native insulin.

In a conjugate according to any one of the previous specific embodiments, the insulin analogue includes the A chain of SEQ ID NO: 3 represented by General Formula 1 below and the B chain of SEQ ID NO: 4 represented by General Formula 2 below (with the proviso that, among the above insulin analogues, those insulin analogues in which the A chain coincides with SEQ ID NO: 1 and simultaneously the B chain coincides with SEQ ID NO: 2 are excluded):
[General Formula 1] 
Xaa1-Xaa2-Val-Glu-Gln-Cys-Cys-Thr-Ser-Ile-Cys-Ser-Leu-Xaa14-Gln-Leu-Glu-As
n-Xaa19-Cys-Asn (SEQ ID NO: 3)
 in which, in General Formula 1 above,
Xaa1 is glycine or alanine;
Xaa2 is isoleucine or alanine;
Xaa14 is tyrosine, glutamic acid, or asparagine; and
Xaa19 is tyrosine or alanine; and
[General Formula 2] 
Phe-Val-Asn-Gln-His-Leu-Cys-Xaa8-Ser-His-Leu-Val-Glu-Ala-Leu-Tyr-Leu-Val-C
ys-Gly-Glu-Arg-Xaa23-Xaa24-Xaa25-Tyr-Thr-Pro-Lys-Thr (SEQ ID NO: 4)
 in General Formula 2 above,
Xaa8 is glycine or alanine;
Xaa23 is glycine or alanine;
Xaa24 is phenylalanine or alanine; and
Xaa25 is phenylalanine or alanine.

In a conjugate according to any one of the previous specific embodiments,
the insulin analogue is characterized in that it includes:
(i) the A chain of General Formula 1 above in which Xaa1 is alanine, Xaa2 is isoleucine, Xaa14 is tyrosine, and Xaa19 is tyrosine, and the B chain of General Formula 2 in which Xaa8 is glycine, Xaa23 is glycine, Xaa24 is phenylalanine, and Xaa25 is phenylalanine;
(ii) the A chain of General Formula 1 in which Xaa1 is glycine, Xaa2 is alanine, Xaa14 is tyrosine, and Xaa19 is tyrosine, and the B chain of General Formula 2 in which Xaa8 is glycine, Xaa23 is glycine, Xaa24 is phenylalanine, and Xaa25 is phenylalanine;
(iii) the A chain of General Formula 1 in which Xaa1 is glycine, Xaa2 is isoleucine, Xaa14 is glutamic acid or asparagine, and Xaa19 is tyrosine, and the B chain of General Formula 2 in which Xaa8 is glycine, Xaa23 is glycine, Xaa24 is phenylalanine, and Xaa25 is phenylalanine;
(iv) the A chain of General Formula 1 in which Xaa1 is glycine, Xaa2 is isoleucine, Xaa14 is tyrosine, and Xaa19 is alanine, and the B chain of General Formula 2 in which Xaa8 is glycine, Xaa23 is glycine, Xaa24 is phenylalanine, and Xaa25 is phenylalanine;
(v) the A chain of General Formula 1 in which Xaa1 is glycine, Xaa2 is isoleucine, Xaa14 is tyrosine, and Xaa19 is tyrosine, and the B chain of General Formula 2 in which Xaa8 is alanine, Xaa23 is glycine, Xaa24 is phenylalanine, and Xaa25 is phenylalanine;
(vi) the A chain of General Formula 1 in which Xaa1 is glycine, Xaa2 is isoleucine, Xaa14 is tyrosine, and Xaa19 is tyrosine, and the B chain of General Formula 2 in which Xaa8 is glycine, Xaa23 is alanine, Xaa24 is phenylalanine, and Xaa25 is phenylalanine;
(vii) the A chain of General Formula 1 in which Xaa1 is glycine, Xaa2 is isoleucine, Xaa14 is tyrosine, and Xaa19 is tyrosine, and the B chain of General Formula 2 in which Xaa8 is glycine, Xaa23 is glycine, Xaa24 is alanine, and Xaa25 is phenylalanine; and
(viii) the A chain of General Formula 1 in which Xaa1 is glycine, Xaa2 is isoleucine, Xaa14 is tyrosine, and Xaa19 is tyrosine, and the B chain of General Formula 2 in which Xaa8 is glycine, Xaa23 is glycine, Xaa24 is phenylalanine, and Xaa25 is alanine.

In a conjugate according to any one of the previous specific embodiments, the insulin analogue is characterized in that it includes the A chain of SEQ ID NO: 5 represented by General Formula 3 below and the B chain of SEQ ID NO: 6 represented by General Formula 4 below (with the proviso that, among the above insulin analogues, those insulin analogues in which the A chain coincides with SEQ ID NO: 1 and simultaneously the B chain coincides with SEQ ID NO: 2 are excluded):
[General Formula 3] 
Xaa1-Ile-Val-Glu-Xaa5-Cys-Cys-Thr-Ser-Ile-Cys-Xaa12-Leu-Xaa14-Gln-Xaa16-G
lu-Asn-Xaa19-Cys-Xaa21 (SEQ ID NO: 5)
 in which, in General Formula 3 above,
Xaa1 is alanine, glycine, glutamine, histidine, glutamic acid, or asparagine,
Xaa5 is alanine, glutamic acid, glutamine, histidine, or asparagine,
Xaa12 is alanine, serine, glutamine, glutamic acid, histidine, or asparagine,
Xaa14 is alanine, tyrosine, glutamic acid, histidine, lysine, aspartic acid, or asparagine,
Xaa16 is alanine, leucine, tyrosine, histidine, glutamic acid, or asparagine,
Xaa19 is alanine, tyrosine, serine, glutamic acid, histidine, threonine, or asparagine, and
Xaa21 is asparagine, glycine, histidine, or alanine; and
[General Formula 4] 
Phe-Val-Asn-Gln-His-Leu-Cys-Gly-Ser-His-Leu-Val-Glu-Ala-Leu-Xaa16-Leu-Val-
Cys-Gly-Glu-Arg-Gly-Phe-Xaa25-Tyr-Xaa27-Xaa28-Lys-Thr (SEQ ID NO: 6)
 in General Formula 4 above,
Xaa16 is tyrosine, glutamic acid, serine, threonine, or aspartic acid, or is absent,
Xaa25 is phenylalanine, aspartic acid, or glutamic acid, or is absent,
Xaa27 is threonine or is absent, and
Xaa28 is proline, glutamic acid, or aspartic acid, or is absent.

In a conjugate according to any one of the previous specific embodiments, the insulin analogue is characterized in that:
in General Formula 3 above, Xaa1 is glycine, Xaa5 is glutamine, Xaa12 is serine, Xaa14 is glutamic acid or asparagine, Xaa16 is leucine, Xaa19 is tyrosine, and Xaa21 is asparagine; and
in General Formula 4 above, Xaa16 is tyrosine, Xaa25 is phenylalanine or is absent, Xaa27 is threonine, Xaa28 is proline, glutamic acid, or aspartic acid, or is absent, but the insulin analogue is not particularly limited thereto.

In a conjugate according to any one of the previous specific embodiments, the insulin analogue is characterized in that:
(1) in the A chain of insulin of SEQ ID NO: 5, Xaa1 is glycine, Xaa5 is glutamine, Xaa12 is serine, Xaa14 is glutamic acid, Xaa16 is leucine, Xaa19 is tyrosine, and Xaa21 is asparagine; and
   in the B chain of insulin of SEQ ID NO: 6, Xaa16 is tyrosine, Xaa25 is phenylalanine, Xaa27 is threonine, Xaa28 is proline, glutamic acid, or aspartic acid, or is absent;
(2) in the A chain of insulin of SEQ ID NO: 5, Xaa1 is glycine, Xaa5 is glutamine, Xaa12 is serine, Xaa14 is asparagine, Xaa16 is leucine, Xaa19 is tyrosine, and Xaa21 is asparagine; and
   in the B chain of insulin of SEQ ID NO: 6, Xaa16 is tyrosine, Xaa25 is phenylalanine, Xaa27 is threonine, Xaa28 is proline, glutamic acid, or aspartic acid, or is absent;
(3) in the A chain of insulin of SEQ ID NO: 5, Xaa1 is glycine, Xaa5 is glutamine, Xaa12 is serine, Xaa14 is glutamic acid, Xaa16 is leucine, Xaa19 is tyrosine, and Xaa21 is asparagine; and,
   in the B chain of insulin of SEQ ID NO: 6, Xaa16 is tyrosine, Xaa25 is absent, Xaa27 is threonine, Xaa28 is proline, glutamic acid, or aspartic acid, or is absent; or
(4) in the A chain of insulin of SEQ ID NO: 5, Xaa1 is glycine, Xaa5 is glutamine, Xaa12 is serine, Xaa14 is alanine, Xaa16 is leucine, Xaa19 is tyrosine, Xaa21 is asparagine; and,
   in the B chain of insulin of SEQ ID NO: 6, Xaa16 is glutamic acid, Xaa25 is absent, Xaa27 is threonine, Xaa28 is proline, glutamic acid, or aspartic acid, or is absent.

In a conjugate according to any one of the previous specific embodiments, the insulin analogue is characterized in that:
(1) in General Formula 3 above, Xaa1 is glycine, Xaa5 is glutamine, Xaa12 is serine, Xaa14 is glutamic acid, Xaa16 is leucine, Xaa19 is tyrosine, and Xaa21 is asparagine; and
   in General Formula 4 above, Xaa16 is tyrosine, Xaa25 is absent, Xaa27 is threonine, and Xaa28 is proline;
(2) in General Formula 3 above, Xaa1 is glycine, Xaa5 is glutamine, Xaa12 is serine, Xaa14 is alanine, Xaa16 is leucine, Xaa19 is tyrosine, and Xaa21 is asparagine; and
   in General Formula 4 above, Xaa16 is glutamic acid, Xaa25 is absent, Xaa27 is threonine, and Xaa28 is proline;
(3) in General Formula 3 above, Xaa1 is glycine, Xaa5 is glutamine, Xaa12 is serine, Xaa14 is histidine, Xaa16 is leucine, Xaa19 is tyrosine, and Xaa21 is asparagine; and
   in General Formula 4 above, Xaa16 is tyrosine, Xaa25 is phenylalanine, Xaa27 is threonine, and Xaa28 is proline;
(4) in General Formula 3 above, Xaa1 is glycine, Xaa5 is glutamine, Xaa12 is serine, Xaa14 is lysine, Xaa16 is leucine, Xaa19 is tyrosine, and Xaa21 is asparagine; and
   in General Formula 4 above, Xaa16 is tyrosine, Xaa25 is phenylalanine, Xaa27 is threonine, and Xaa28 is proline;
(5) in General Formula 3 above, Xaa1 is glycine, Xaa5 is glutamine, Xaa12 is serine, Xaa14 is tyrosine, Xaa16 is leucine, Xaa19 is glutamic acid, and Xaa21 is asparagine; and
   in General Formula 4 above, Xaa16 is tyrosine, Xaa25 is phenylalanine, Xaa27 is threonine, and Xaa28 is proline;
(6) in General Formula 3 above, Xaa1 is glycine, Xaa5 is glutamine, Xaa12 is serine, Xaa14 is tyrosine, Xaa16 is leucine, Xaa19 is serine, and Xaa21 is asparagine; and
   in General Formula 4 above, Xaa16 is tyrosine, Xaa25 is phenylalanine, Xaa27 is threonine, and Xaa28 is proline;
(7) in General Formula 3 above, Xaa1 is glycine, Xaa5 is glutamine, Xaa12 is serine, Xaa14 is tyrosine, Xaa16 is leucine, Xaa19 is threonine, and Xaa21 is asparagine; and
   in General Formula 4 above, Xaa16 is tyrosine, Xaa25 is phenylalanine, Xaa27 is threonine, and Xaa28 is proline;
(8) in General Formula 3 above, Xaal is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is glutamic acid, Xaa25 is phenylalanine, Xaa27 is threonine, and Xaa28 is proline;
(9) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is serine, Xaa25 is phenylalanine, Xaa27 is threonine, and Xaa28 is proline;
(10) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is threonine, Xaa25 is phenylalanine, Xaa27 is threonine, and Xaa28 is proline;
(11) in General Formula 3 above, Xaa1 is glycine, Xaa5 is glutamine, Xaa12 is serine, Xaa14 is alanine, Xaa16 is leucine, Xaa19 is tyrosine, and Xaa21 is asparagine; and
   in General Formula 4 above, Xaa16 is tyrosine, Xaa25 is phenylalanine, Xaa27 is threonine, and Xaa28 is proline;
(12) in General Formula 3 above, Xaa1 is glycine, Xaa5 is glutamine, Xaa12 is serine, Xaa14 is aspartic acid, Xaa16 is leucine, Xaa19 is tyrosine, and Xaa21 is asparagine; and
   in General Formula 4 above, Xaa16 is tyrosine, Xaa25 is phenylalanine, Xaa27 is threonine, and Xaa28 is proline;
(13) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is aspartic acid, Xaa25 is phenylalanine, Xaa27 is threonine, and Xaa28 is proline;
(14) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is tyrosine, Xaa25 is aspartic acid, Xaa27 is threonine, and Xaa28 is proline; or
(15) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is tyrosine, Xaa25 is glutamic acid, Xaa27 is threonine, and Xaa28 is proline.

In a conjugate according to any one of the previous specific embodiments, the conjugate is represented by Formula 2 below:

[Formula 2] X-L-F

in which in Formula 2 above,
X is native insulin or an insulin analogue with a reduced binding affinity for an insulin receptor compared to native insulin;
L, being a linker, is polyethylene glycol having a size of greater than 0 kDa to less than 3.4 kDa; and
F is a material capable increasing *in vivo* half-life of X.

In a conjugate according to any one of the previous specific embodiments, the conjugate represented by Formula 2 above has reduced receptor-mediated clearance (RMC).

In a conjugate according to any one of the previous specific embodiments, in Formula 2 above, L is linked to an amino terminal region of beta chain of X, which is native insulin or an analogue thereof.

In a conjugate according to any one of the previous specific embodiments, X and F are linked to each other through L by a covalent chemical bond, a non-covalent chemical bond, or a combination thereof.

In a conjugate according to any one of the previous specific embodiments, the material capable of increasing *in vivo* half-life of the physiologically active material is a biocompatible material.

In a conjugate according to any one of the previous specific embodiments, F is selected from the group consisting of polymers, fatty acids, cholesterol, albumin and a fragment thereof, albumin-binding materials, a polymer of repeating units of a particular amino acid sequence, antibodies, antibody fragments, FcRn-binding materials, *in vivo* connective tissues, nucleotides, fibronectin, transferrin, saccharides, heparin, and elastin.

In a conjugate according to any one of the previous specific embodiments, the polymer is selected from the group consisting of polyethylene glycol, polypropylene glycol, an ethylene glycol-propylene glycol copolymer, polyoxyethylated polyol, polyvinyl alcohol, a polysaccharide, dextran, polyvinyl ethyl ether, a biodegradable polymer, a lipid polymer, chitins, hyaluronic acid, an oligonucleotide, and a combination thereof.

In a conjugate according to any one of the previous specific embodiments, the FcRn-binding material is a polypeptide including an immunoglobulin Fc region.

In a conjugate according to any one of the previous specific embodiments, F is an IgG Fc region.

In a conjugate according to any one of the previous specific embodiments, in Formula 1 or Formula 2 above, F is an IgG Fc region and L is linked to the N-terminal region of F.

Another aspect of the present invention provides a method for preparing the conjugate.

In a specific embodiment, the present invention relates to a method for preparing a conjugate, which includes:
(a) reacting polyethylene glycol, which has a size of greater than 0 kDa to less than 3.4 kDa and at least two terminal functional groups, with any one of a physiologically active material or a material capable of increasing *in vivo* half-life of the physiologically active material to prepare polyethylene glycol, to which one of the physiologically active material or the material capable of increasing *in vivo* half-life of the physiologically active material is covalently linked and which has at least one terminal functional group; and
(b) reacting the polyethylene glycol, to which one of the physiologically active material or the material capable of increasing *in vivo* half-life of the physiologically active material is covalently linked and which has at least one terminal functional group, prepared in step (a) with the other of a physiologically active material or a material capable of increasing *in vivo* half-life of the physiologically active material to prepare a conjugate in which the physiologically active material and a material capable of increasing *in vivo* half-life of the physiologically active material are covalently linked through polyethylene glycol having a size of greater than 0 kDa to less than 3.4 kDa.

In a method according to the previous specific embodiment, the physiologically active material has a functional group which forms a covalent bond by reacting with a terminal functional group of polyethylene glycol, and the functional group is an amine group or thiol group.

In a method according to any one of the previous specific embodiments, the material capable of increasing *in vivo* half-life of a physiologically active material has a functional group which forms a covalent bond by reacting with a terminal functional group of polyethylene glycol, and the functional group is an amine group or thiol group.

In a method according to any one of the previous specific embodiments, the terminal functional group of polyethylene glycol is an amine-reactive functional group or thiol-reactive functional group.

In a method according to any one of the previous specific embodiments, the terminal functional group of polyethylene glycol is selected from the group consisting of aldehyde, maleimide, succinimide, vinylsulfone, thiol, C₆₋₂₀ aryl disulfide, C₅₋₂₀ heteroaryl disulfide, and halogenated acetamide.

In a method according to any one of the previous specific embodiments, the terminal functional group of polyethylene glycol is selected from the group consisting of aldehyde, maleimide, succinimide, vinylsulfone, thiol, *ortho*-pyridyl disulfide, and iodoacetamide.

In a method according to any one of the previous specific embodiments, the succinimide is succinimidyl valerate, succinimidyl methylbutanoate, succinimidyl methylpropionate, succinimidyl butanoate, succinimidyl propionate, *N*-hydroxysuccinimide, succinimidyl carboxymethyl, or succinimidyl carbonate.

Still another aspect of the present invention relates to a long-acting insulin preparation with *in vivo* duration and stability containing the conjugate.

Still another aspect of the present invention relates to a preparation for preventing or treating diabetes containing the conjugate.

Still another aspect of the present invention relates to a method for treating insulin-related diseases including administering the conjugate represented by Formula 2 or a composition or a preparation containing the conjugate to a subject in need thereof.

### [Advantageous Effects]

The conjugate of the physiologically active material according to the present invention can be effectively used in fields where the corresponding physiological activity is necessary.

### [Brief Description of Drawings]

FIG. 1 shows the results of SDS-PAGE with respect to an insulin-3.4 kDa PEG-immunoglobulin Fc fragment conjugate.
FIG. 2 shows the results of SDS-PAGE with respect to an insulin-1 kDa PEG-immunoglobulin Fc fragment conjugate.
FIG. 3 shows the results of size-exclusion high performance liquid chromatography (SE-HPLC) with respect to each of insulin-1, 2, 2.5, 3, 3.4 kDa PEG-immunoglobulin Fc fragment conjugates.
FIG. 4 shows the results of reverse-phase high performance liquid chromatography (RP-HPLC) with respect to each of insulin-1, 2, 2.5, 3, 3.4 kDa PEG-immunoglobulin Fc fragment conjugates.

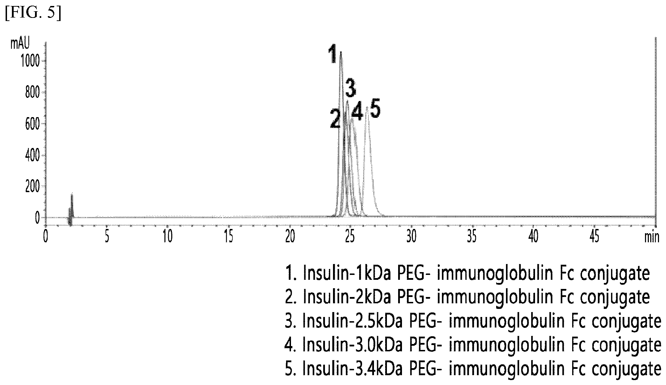

FIG. 5 shows the graph of chromatography overlay with respect to insulin-1, 2, 2.5, 3, 3.4 kDa PEG-immunoglobulin Fc fragment conjugates.
FIG. 6 shows the comparison results of duration of efficacy with respect to long-acting insulin conjugates according to the length of a PEG linker.

### [Best Mode]

Hereinafter, the present invention will be described in more detail.

Meanwhile, each of the explanations and exemplary embodiments disclosed herein can be applied to their respective other explanations and exemplary embodiments. That is, all of the combinations of various factors disclosed herein belong to the scope of the present invention. Additionally, the scope of the present invention should not be limited by the specific disclosure provided hereinbelow. Additionally, an ordinary person skilled in the art will be able to recognize or confirm using no more than routine experimentation with respect to a number of equivalents to the specific embodiments of the invention described in the present invention. Additionally, such equivalents are intended to be included in the present invention.

Throughout the specification, the conventional one-letter and three-letter codes for amino acids are used. Additionally, the amino acids mentioned herein are abbreviated according to the nomenclature rules of IUPAC-IUB.

An aspect of the present invention provides a conjugate of a physiologically active material for the purpose of extending *in vivo* half-life of the physiologically active material.

Specifically, the present invention provides a conjugate of a physiologically active material, in which a physiologically active material and a material capable of increasing *in vivo* half-life of the physiologically active material are linked through polyethylene glycol, and the polyethylene glycol has a size of greater than 0 kDa to less than 3.4 kDa.

In a more specific embodiment, the present invention provides a conjugate of Formula 1 below:

[Formula 1] X-L-F

in which
X is a physiologically active material;
L, being a linker, is polyethylene glycol having a size of greater than 0 kDa to less than 3.4 kDa; and
F is a material capable increasing *in vivo* half-life of the physiologically active material.

As used herein, the term "conjugate represented by Formula 1 above" is interchangeably used with "long-acting conjugate".

As used herein, the term "long-acting conjugate" refers to a physiologically active material which is linked to a material which is capable of extending *in vivo* half-life of the physiologically active material, e.g., a biocompatible material, through a linker.

The conjugate may exhibit an increased *in vivo* half-life compared to a conjugate which only differs from the conjugate in that L is polyethylene glycol having a size of 3.4 kDa, but the conjugate is not particularly limited thereto.

More specifically, the conjugate may exhibit a reduced binding affinity for a receptor of the physiologically active material compared to a conjugate which has the same X and F as the conjugate but has a different L, which is polyethylene glycol having a size of 3.4 kDa, and may exhibit an increased blood half-life due to the weakening of receptor-mediated clearance (RMC), but the conjugate is not particularly limited thereto.

Additionally, further to the above effects or independently thereof, the conjugate may exhibit substantially the same activity as or greater activity with respect to the physiological activity of X itself or the activity of F itself compared to a conjugate which has the same X and F as the conjugate but has a different L, which is polyethylene glycol having a size of 3.4 kDa, even if X and F are present in close proximity due to polyethylene glycol that links between X and F and has a size less than 3.4 kDa, but the conjugate is not particularly limited thereto.

In the present invention, L, being a linker and a moiety constituting the conjugate, refers to polyethylene glycol having a size of greater than 0 kDa to less than 3.4 kDa, and it includes all of the polyethylene glycols having a size of greater than 0 kDa to less than 3.4 kDa, greater than 0 kDa to 3.3 kDa or less, greater than 0 kDa to 3.2 kDa or less, greater than 0 kDa to 3.1 kDa or less, greater than 0 kDa to 3.0 kDa or less, greater than 0 kDa to 2.9 kDa or less, greater than 0 kDa to 2.8 kDa or less, greater than 0 kDa to 2.7 kDa or less, greater than 0 kDa to 2.6 kDa or less, greater than 0 kDa to 2.5 kDa or less, greater than 0 kDa to 2.4 kDa or less, greater than 0 kDa to 2.3 kDa or less, greater than 0 kDa to 2.2 kDa or less, greater than 0 kDa to 2.1 kDa or less, greater than 0 kDa to 2.0 kDa or less, greater than 0 kDa to 1.9 kDa or less, greater than 0 kDa to 1.8 kDa or less, greater than 0 kDa to 1.7 kDa or less, greater than 0 kDa to 1.6 kDa or less, greater than 0 kDa to 1.5 kDa or less, greater than 0 kDa to 1.4 kDa or less, greater than 0 kDa to 1.3 kDa or less, greater than 0 kDa to 1.2 kDa or less, greater than 0 kDa to 1.1 kDa or less, greater than 0 kDa to 1.0 kDa or less, equal to or greater than 0.5 kDa to less than 3.4 kDa, equal to or greater than 0.5 kDa to 3.3 kDa or less, equal to or greater than 0.5 kDa to 3.2 kDa or less, equal to or greater than 0.5 kDa to 3.1 kDa or less, equal to or greater than 0.5 kDa to 3 kDa or less, equal to or greater than 0.5 kDa to 2.9 kDa or less, equal to or greater than 0.5 kDa to 2.8 kDa or less, equal to or greater than 0.5 kDa to 2.7 kDa or less, equal to or greater than 0.5 kDa to 2.6 kDa or less, equal to or greater than 0.5 kDa to 2.5 kDa or less, equal to or greater than 0.5 kDa to 2.4 kDa or less, equal to or greater than 0.5 kDa to 2.3 kDa or less, equal to or greater than 0.5 kDa to 2.2 kDa or less, equal to or greater than 0.5 kDa to 2.1 kDa or less, equal to or greater than 0.5 kDa to 2 kDa or less, equal to or greater than 0.5 kDa to 1.9 kDa or less, equal to or greater than 0.5 kDa to 1.8 kDa or less, equal to or greater than 0.5 kDa to 1.7 kDa or less, equal to or greater than 0.5 kDa to 1.6 kDa or less, equal to or greater than 0.5 kDa to 1.5 kDa or less, equal to or greater than 0.5 kDa to 1.4 kDa or less, equal to or greater than 0.5 kDa to 1.3 kDa or less, equal to or greater than 0.5 kDa to 1.2 kDa or less, equal to or greater than 0.5 kDa to 1.1 kDa or less, equal to or greater than 0.5 kDa to 1 kDa or less, greater than 0.5 kDa to less than 3.4 kDa, greater than 0.5 kDa to 3.3 kDa or less, greater than 0.5 kDa to 3.2 kDa or less, greater than 0.5 kDa to 3.1 kDa or less, greater than 0.5 kDa to 3 kDa or less, greater than 0.5 kDa to 2.9 kDa or less, greater than 0.5 kDa to 2.8 kDa or less, greater than 0.5 kDa to 2.7 kDa or less, greater than 0.5 kDa to 2.6 kDa or less, greater than 0.5 kDa to 2.5 kDa or less, greater than 0.5 kDa to 2.4 kDa or less, greater than 0.5 kDa to 2.3 kDa or less, greater than 0.5 kDa to 2.2 kDa or less, greater than 0.5 kDa to 2.1 kDa or less, greater than 0.5 kDa to 2 kDa or less, greater than 0.5 kDa to 1.9 kDa or less, greater than 0.5 kDa to 1.8 kDa or less, greater than 0.5 kDa to 1.7 kDa or less, greater than 0.5 kDa to 1.6 kDa or less, greater than 0.5 kDa to 1.5 kDa or less, greater than 0.5 kDa to 1.4 kDa or less, greater than 0.5 kDa to 1.3 kDa or less, greater than 0.5 kDa to 1.2 kDa or less, greater than 0.5 kDa to 1.1 kDa or less, greater than 0.5 kDa to 1.0 kDa or less, equal to or greater than 0.75 kDa to less than 3.4 kDa, equal to or greater than 0.75 kDa to 3.3 kDa or less, equal to or greater than 0.75 kDa to 3.2 kDa or less, equal to or greater than 0.75 kDa to 3.1 kDa or less, equal to or greater than 0.75 kDa to 3 kDa or less, equal to or greater than 0.75 kDa to 2.9 kDa or less, equal to or greater than 0.75 kDa to 2.8 kDa or less, equal to or greater than 0.75 kDa to 2.7 kDa or less, equal to or greater than 0.75 kDa to 2.6 kDa or less, equal to or greater than 0.75 kDa to 2.5 kDa or less, equal to or greater than 0.75 kDa to 2.4 kDa or less, equal to or greater than 0.75 kDa to 2.3 kDa or less, equal to or greater than 0.75 kDa to 2.2 kDa or less, equal to or greater than 0.75 kDa to 2.1 kDa or less, equal to or greater than 0.75 kDa to 2 kDa or less, equal to or greater than 0.75 kDa to 1.9 kDa or less, equal to or greater than 0.75 kDa to 1.8 kDa or less, equal to or greater than 0.75 kDa to 1.7 kDa or less, equal to or greater than 0.75 kDa to 1.6 kDa or less, equal to or greater than 0.75 kDa to 1.5 kDa or less, equal to or greater than 0.75 kDa to 1.4 kDa or less, equal to or greater than 0.75 kDa to 1.3 kDa or less, equal to or greater than 0.75 kDa to 1.2 kDa or less, equal to or greater than 0.75 kDa to 1.1 kDa or less, equal to or greater than 0.75 kDa to 1 kDa or less, greater than 0.75 kDa to 2 kDa or less, greater than 0.75 kDa to less than 2 kDa, greater than 0.75 kDa to 1.5 kDa or less, about 1.0 kDa, or 1.0 kDa, but the size of polyethylene glycol is not limited thereto.

As used herein, the term "about" refers to a range which includes all of ±0.5, ±0.4, ±0.3, ±0.2, ±0.1, *etc.,* and includes all of the values that are equivalent or similar to those following the values, but the range is not limited thereto.

As used herein, the term "X" refers to a physiologically active material that is a moiety constituting the conjugate. The physiologically active material refers to a material that has any physiological activity *in vivo.*

The physiologically active material may be toxins or physiologically active polypeptides, and may include various kinds of physiologically active polypeptides used for the treatment or prevention of human diseases, such as cytokines, interleukins, interleukin-binding proteins, enzymes, antibodies, growth factors, transcription control factors, blood factors, vaccines, structural proteins, ligand proteins or receptors, cell surface antigens, receptor antagonists, physiologically active peptides released in the small intestine and pancreas having therapeutic effects on the treatment of diabetes and obesity, G protein-coupled receptors (GPCR) agonists or antagonists, *etc.,* or analogues thereof, but the physiologically active material is not limited thereto.

As used herein, the term "analogue of X" refers to a material capable of exhibiting the same kind of activity as X, and it includes all of the agonists of X, derivatives of X, fragments of X, variants of X, *etc.*

Such X may be a native physiologically active polypeptide.

Specifically, the "derivative of a native physiologically active polypeptide" includes peptides which have at least one difference in the amino acid sequence compared to that of a native physiologically active polypeptide; modified peptides prepared by modification of the sequence of a native physiologically active polypeptide; and mimetics having the same kind of activity as a native physiologically active polypeptide.

Specifically, the derivative of a native physiologically active polypeptide may be prepared by using any one method selected from substitution, addition, deletion, modification, and a combination thereof with respect to a part of the amino acids of a native physiologically active polypeptide, and artificial peptides, which were manipulated to have a binding affinity for at least two mutually different receptors prepared by such method, also belong to the scope of the above derivative.

Additionally, the modification for the preparation of the derivative of a native physiologically active polypeptide includes all of a modification using an L-type or D-type amino acid, and/or a non-native amino acid; and/or a modification of a native sequence (e.g., modification of functional group(s) in a side chain, an intramolecular covalent bonding (e.g., ring formation between side chains, methylation, acylation, ubiquitination, phosphorylation, amino-hexanation, biotinylation, *etc.*))*.*

Additionally, the derivative of a native physiologically active polypeptide also includes those in which one or more amino acids are added to the amino- and/or carboxy-terminus of a native physiologically active polypeptide.

As the amino acids to be substituted or added, atypical or non-naturally occurring amino acids as well as the 20 amino acids conventionally observed in human proteins may be used.

As used herein, the term "fragment of a native physiologically active polypeptide or fragment of a derivative of a native physiologically active polypeptide" refers to a form of a native physiologically active polypeptide or a derivative of a native physiologically active polypeptide in which one or more amino acids are deleted from the amino- or carboxy-terminus of a native physiologically active polypeptide or a derivative of a native physiologically active polypeptide.

The physiologically active material may be selected from the group consisting of toxins; glucagon-like peptide-1 (GLP-1) receptor agonists; glucagon receptor agonists; gastric inhibitory polypeptide (GIP) receptor agonists; fibroblast growth factor (FGF) receptor agonists; cholecystokinin receptor agonists; gastrin receptor agonists (gastrin); melanocortin receptor agonists; materials binding to two or more receptors among a GLP receptor, a glucagon receptor, and a GIP receptor; somatostatin; peptide YY (PYY); neuropeptide Y (NPY); oxyntomodulin; fibroblast growth factor (FGF); bradykinin; eledoisin; oxytocin; vasopressin; sermorelin; prolactin-releasing peptides; orexin; thyroid-releasing peptides; calmodulin; motilin; vasoactive intestinal peptides; atrial natriuretic peptides (ANP); C-type natriuretic peptides (CNP); neurokinin A; neuromedin; renin; endothelin; sarafotoxin peptides; carsomorphin peptides; dermorphin; dynorphin; endorphin; enkepalin; tumor necrosis factor receptors; urokinase receptors; thymopoietin; thymulin; thymopentin; tymosin; thymic humoral factors; adrenomodullin; allatostatin; amyloid β-protein fragments; antibiotic peptides; antioxidant peptides; bombesin; osteocalcin; CART peptides; E-selectin; intercellular adhesion molecule 1 (ICAM-1); vascular cell adhesion molecule 1 (VCAM-1); leucokine; kringle-5; laminin; inhibin; galanin; fibronectin; pancreastatin; fuzeon; glucagon-like peptides; G protein-coupled receptors; erythropoietic growth factors; leukopoietin; amylin; human growth hormone; growth hormone-releasing hormone; growth hormone-releasing peptides; interferons; interferon receptors; colony-stimulating factors; interleukins; interleukin receptors; enzymes; interleukin-binding proteins; cytokine-binding proteins; macrophage-activating factors; macrophage peptides; B cell factors; T cell factors; protein A; allergy-inhibiting factors; necrosis glycoproteins; immunotoxins; lymphotoxins; tumor necrosis factors; tumor suppressors; transforming growth factors; α-1 antitrypsin; albumin; α-lactalbumin; apolipoprotein-E; erythropoietin; high-glycosylated erythropoietin; angiopoietins; hemoglobins; thrombin; thrombin receptor-activating peptides; thrombomodulin; blood factor VII (blood coagulation factor VII); blood factor VIIa (blood coagulation factor VIIa); blood factor VIII (blood coagulation factor VIII); blood factor IX (blood coagulation factor IX); blood factor XIII (blood coagulation factor XIII); plasminogen activators; fibrin-binding peptides; urokinase; streptokinase; hirudin; protein C; C-reactive protein; renin inhibitors; collagenase inhibitors; superoxide dismutase; leptin; platelet-derived growth factor; epithelial growth factor; epidermal growth factor; angiostatin; angiotensin; bone morphogenetic growth factor; bone morphogenetic protein; calcitonin; insulin; atriopeptin; cartilage-inducing factor; elcatonin; connective tissue-activating factor; tissue factor pathway inhibitor; follicle-stimulating hormone; luteinizing hormone; luteinizing hormone-releasing hormone; nerve growth factors; axogenesis factor-1; brain-natriuretic peptide; glial-derived neurotrophic factor; netrin; neutrophil inhibitory factor; neurotrophic factor; neurturin; parathyroid hormone; relaxin; secretin; somatomedin; insulin-like growth factor; adrenocortical hormone; glucagon; cholecystokinin; pancreatic polypeptides; gastrin-releasing peptides; gastrin inhibitory peptides; corticotropin-releasing factor; thyroid-stimulating hormone; autotaxin; lactoferrin; myostatin; activity-dependent neuroprotective protein (ADNP), β-secretase1 (BACE1), amyloid precursor protein (APP), neural cell adhesion molecule (NCAM), amyloid β, tau, receptor for advanced glycation endproducts (RAGE), α-synuclein, or agonists or antagonists thereof; receptors, receptor agonists; cell surface antigens; monoclonal antibody; polyclonal antibody; antibody fragments; virus-derived vaccine antigens; hybrid polypeptides or chimeric polypeptides that activate at least one receptor agonist; and analogues thereof.

Additionally, the toxin may be selected from the group consisting of maytansine or a derivative thereof, auristatin or a derivative thereof, duocarmycin or a derivative thereof, and pyrrolobenzodiazepine (PBD) or a derivative thereof;
the glucagon-like peptide-1 (GLP-1) receptor agonist may be selected from the group consisting of native glucagon-like peptide-1 (GLP-1), native GLP-2, native exendin-3, native exendin-4, and analogues thereof;
the FGF receptor agonist may be selected from the group consisting of FGF1 or an analogue thereof, FGF19 or an analogue thereof, FGF21 or an analogue thereof, and FGF23 or an analogue thereof;
the interferon may be selected from the group consisting of interferon-a, interferon-β, and interferon-y;
the interferon receptor may be selected from the group consisting of interferon-a receptor, interferon-β receptor, interferon-y receptor, and soluble type I interferon receptors;
the interleukin is selected from the group consisting of interleukin-1, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18, interleukin-19, interleukin-20, interleukin-21, interleukin-22, interleukin-23, interleukin-24, interleukin-25, interleukin-26, interleukin-27, interleukin-28, interleukin-29, and interleukin-30;
the interleukin receptor may be interleukin-1 receptor or interleukin-4 receptor;
the enzyme may be selected from the group consisting of β-glucosidase, α-galactosidase, β-galactosidase, iduronidase, iduronate-2-sulfatase, galactose-6-sulfatase, acid α-glucosidase, acid ceramidase, acid sphingomyelinase, galactocerebrosidase, arylsulfatase A, arylsulfatase B, β-hexosaminidase A, β-hexosaminidase B, heparin N-sulfatase, α-D-mannosidase, β-glucuronidase, N-acetylgalactosamine-6 sulfatase, lysosomal acid lipase, α-*N*-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, myeloperoxidase, α-galactosidase-A, agalsidase α, agalsidase β, α-L-iduronidase, butyrylcholinesterase, chitinase, glutamate decarboxylase, and imiglucerase;
the interleukin-binding protein may be IL-18bp;
the cytokine-binding protein may be TNF-binding protein;
the nerve growth factors may be selected from the group consisting of nerve growth factor, ciliary neurotrophic factor, axogenesis factor-1, brain-natriuretic peptide, glial-derived neurotrophic factor, netrin, neutrophil inhibitory factor, neurotrophic factor, and neurturin;
the myostatin receptor may be selected from the group consisting of TNFR (P75), TNFR (P55), IL-1 receptor, VEGF receptor, and B cell activating factor receptor;
the myostatin receptor antagonist may be IL1-Ra;
the cell surface antigen may be selected from the group consisting of CD2, CD3, CD4, CD5, CD7, CD11a, CD11b, CD18, CD19, CD20, CD23, CD25, CD33, CD38, CD40, CD45, and CD69; and
the antibody fragments may be selected from the group consisting of scFv, Fab, Fab', F(ab')₂, and Fd, but these physiologically active materials are not particularly limited thereto.

Additionally, the physiologically active material may be selected from the group consisting of native exendin-3 or an analogue thereof; native exendin-4 or an analogue thereof; native insulin or an analogue thereof; native GLP-1 or an analogue thereof; native GLP-2 or an analogue thereof; native oxyntomodulin or an analogue thereof; native glucagon or an analogue thereof; native fibroblast growth factor or an analogue thereof; native ghrelin or an analogue thereof; native calcitonin or an analogue thereof; and native granulocyte-colony stimulating factor or an analogue thereof, but the physiologically active material is not particularly limited thereto.

Specifically, as the physiologically active material, the native exendin-3 analogue may be selected from the group consisting of exendin-3 in which the N-terminal amine group is deleted from native exendin-3; exendin-3 in which the N-terminal amine group of native exendin-3 is substituted with a hydroxyl group; exendin-3 in which the N-terminal amine group of native exendin-3 is modified with a dimethyl group; exendin-3 in which the N-terminal amine group of native exendin-3 is substituted with a carboxyl group; and exendin-3 in which the α-carbon of the 1^{st} amino acid of native exendin-3 (*i.e.,* histidine) is deleted from native exendin-3; and exendin-3 in which the 12^{th} amino acid of the exendin-3 *(i.e.,* lysine) is substituted with serine; and exendin-3 in which the 12^{th} amino acid of the exendin-3 *(i.e.,* lysine) is substituted with arginine; and
as the physiologically active material, the native exendin-4 analogue may be selected from the group consisting of exendin-4 in which the N-terminal amine group is deleted from native exendin-4; exendin-4 in which the N-terminal amine group of native exendin-4 is substituted with a hydroxyl group; exendin-4 in which the N-terminal amine group of native exendin-4 is modified with a dimethyl group; exendin-4 in which the N-terminal amine group of native exendin-4 is substituted with a carboxyl group; and exendin-4 in which the α-carbon of the 1^{st} amino acid of native exendin-4 (*i.e.,* histidine) is deleted from native exendin-4; and exendin-4 in which the 12^{th} amino acid of the exendin-4 *(i.e.,* lysine) is substituted with serine; and exendin-4 in which the 12^{th} amino acid of the exendin-4 (*i.e.,* lysine) is substituted with arginine, but the native exendin-3 analogue and native exendin-4 analogue are not particularly limited thereto.

More specifically, the native exendin-4 analogue may be one selected from the group consisting of des-amino-histidyl(DA)-exendin-4, β-hydroxy-imidazo-propionyl(HY)-exendin-4, imidazo-acetyl(CA)-exendin-4, and dimethyl-histidyl(DM)-exendin-4, but the native exendin-4 analogue is not particularly limited thereto.

The derivatives of native exendin that belong to the scope of the above exendins are described in detail in Korean Patent Application Publication No. 10-2009-0008151 (International Patent Publication No. WO 2009/011544 A2). Additionally, the entire specification of the Korean Patent Application Publication (International Patent Publication) is incorporated herein by reference.

Additionally, the physiologically active material may be native oxyntomodulin or a derivative thereof. The derivatives of native oxyntomodulin that belong to the scope of the oxyntomodulin are described in detail in Korean Patent Application Publication No. 10-2012-0139579 (International Patent Publication No. WO 2012/173422 A9) and Korean Patent Application Publication No. 10-2012-0137271 (International Patent Publication No. WO 2012/169798 A2). Additionally, the entire specifications of the Korean Patent Application Publications (International Patent Publications) are incorporated herein by reference.

Additionally, the physiologically active material may be native granulocyte-colony stimulating factor or a derivative thereof.

Specifically, the physiologically active material may be native human granulocyte-colony stimulating factor derivative, in which any one of the 1^{st}, the 2^{nd}, the 3^{rd}, and the 17^{th} amino acid of native human granulocyte-colony stimulating factor is substituted with a different amino acid, and the 65^{th} amino acid residue *(i.e.,* proline) is further substituted with serine. More specifically, the physiologically active material may be a derivative of native human granulocyte-colony stimulating factor which includes those where the 17^{th} amino acid residue (*i.e.,* cysteine), the 65^{th} amino acid residue (*i.e.,* proline), and both of these amino acid residues are substituted with serine, but the physiologically active material is not particularly limited thereto.

The derivatives of native granulocyte-colony stimulating factor that belong to the scope of the granulocyte-colony stimulating factor are described in detail in Korean Patent Application Publication No. 10-2001-0009171 (International Patent Publication No. WO 2001/004329 A1). Additionally, the entire specification of the Korean Patent Application Publication (International Patent Publication) is incorporated herein by reference.

Additionally, the physiologically active material may be a material that can bind to two or more receptors among a GLP receptor, a glucagon receptor, and a GIP receptor. The material may have an activity for two or more receptors among the GLP receptor, glucagon receptor, and GIP receptor. That is, once a material binds to a subject receptor, the receptor can exhibit activity.

Specifically, the physiologically active material may be a material that can bind to two or more receptors among a glucagon receptor, a GLP-1 receptor, and a GIP receptor, and more specifically, a material that can bind to a glucagon receptor and a GLP-1 receptor; a material that can bind to a GLP-1 receptor and a GIP receptor; a material that can bind to a GLP-1 receptor and a glucagon receptors; or a material that can bind to all of a glucagon receptor, a GLP-1 receptor, and a GIP receptor, but the physiologically active material is not particularly limited thereto. The material which can bind to three receptors may be named a triple agonist (or a triple activator) and the material which can bind to two receptors may be named a dual agonist.

Examples of the peptides having an activity for a glucagon receptor, a GLP-1 receptor, and a GIP receptor are described in WO 2017/116205 A1 and WO 2017/116204 A1, which are applications previously filed by the applicant of the present invention, and the entirety of these applications are incorporated herein by references.

Additionally, examples of the glucagon/GLP-1 dual agonist are described in WO 2015/183054, WO 2016/043533, *etc.* and the entire specifications of these applications are incorporated herein by reference.

Additionally, the physiologically active material may be a derivative of native glucagon.

Examples of the glucagon derivative are described in WO 2016/108586, WO 2017/003191, *etc.* and the entire specifications of these applications are incorporated herein by reference.

Additionally, the physiologically active material may be native insulin or an analogue thereof. More specifically, the physiologically active material may be native insulin or an insulin analogue with a reduced binding affinity for an insulin receptor compared to native insulin.

As used herein, the term "insulin analogue" refers to non-native insulin which is different from native insulin. The insulin analogue includes non-native human insulin which is different from native human insulin.

Such insulin analogue includes those insulin analogues in which part of the amino acids of native insulin is modified in the form of addition and/or deletion and/or substitution.

Specifically, the insulin analogue of the present invention may have a sequence homology to the sequence of native insulin of at least 60%, at least 65%, at least 70%, at least 75%, at least 80%, at least 85%, at least 90%, at least 91%, at least 92%, at least 93%, at least 94%, or at least 95% when comparing sequence identity. Additionally, the insulin analogue of the present invention may be those having a reduced receptor binding affinity compared to native insulin while having the above sequence homology to that of native insulin. Additionally, the insulin analogue may be those which have glucose uptake ability and/or have blood glucose-lowering ability *in vivo* as in native insulin.

More specifically, the insulin analogue of the present invention may exhibit binding affinity for an insulin receptor of about 99% or less, about 95% or less, about 90% or less, about 85% or less, about 80% or less, about 75% or less, about 70% or less, about 65% or less, about 60% or less, about 55% or less, about 50% or less, about 45% or less, about 40% or less, about 35% or less, about 30% or less, about 25% or less, about 20% or less, about 15% or less, about 10% or less, about 9% or less, about 8% or less, about 7% or less, about 6% or less, about 5% or less, about 4% or less, about 3% or less, about 2% or less, about 1% or less, or about 0.1% or less, compared to the binding affinity (100%) of native insulin (but the binding affinity of the insulin analogue of the present invention for an insulin receptor does not correspond to 0%). The binding affinity of the insulin analogue may be evaluated using the Scintillation Proximity Assay (SPA) which utilizes the competitive reaction between insulin analogues and I¹²⁵-bound insulin in a cell membrane that overexpresses recombinant human insulin receptors.

Additionally, the insulin analogue may be those which have an increased half-life by at least 10% compared to native insulin due to the decrease of binding affinity for an insulin receptor, but the insulin analogue is not limited thereto.

Additionally, the insulin analogue of the present invention may have glucose uptake ability as in native insulin.

Specifically, the insulin analogue of the present invention may be those which have glucose uptake ability of at least about 10%, at least about 20%, at least about 30%, at least about 40%, at least about 50%, at least about 55%, at least about 60%, at least about 65%, at least about 70%, at least about 75%, at least about 80%, at least about 85%, at least about 90%, at least about 95%, at least about 100%, at least about 110%, at least about 120%, at least about 130%, at least about 140%, at least about 150%, at least about 160%, at least about 170%, at least about 180%, at least about 190%, or at least about 200%, compared to the glucose uptake ability (100%) of native insulin.

The measurement of glucose uptake ability can be achieved using various methods for measuring glucose uptake ability known in the art.

The insulin analogue that belongs to the scope of the above insulin analogue are described in detail in Korean Patent Application Publication No. 10-2015-0087130 (International Patent Publication No. WO 2015/108398 A1) and Korean Patent Application Publication No. 10-2017-0026284 (International Patent Publication No. WO 2017/039267 A1). Additionally, the entire specifications of the Korean Patent Application Publications (International Patent Publications) are incorporated herein by reference, but the insulin analogue is not limited thereto. Additionally, the insulin analogue of the present invention also includes all of those insulin analogues disclosed in Korean Patent Application Publication No. 10-2014-0106452, but the insulin analogue is not limited thereto. All of the above patent specifications are incorporated herein by reference.

Specifically, the insulin analogue may be in the form of a single polypeptide chain or two polypeptide chains, and more preferably two polypeptide chains, but the insulin analogue is not particularly limited thereto.

The insulin analogue in the form of two polypeptide chains may be those which consist of a polypeptide corresponding to the A chain of native insulin and a polypeptide corresponding to the B chain of native insulin. In particular, the "corresponding to the A chain or B chain of native insulin" may refer to cases in which any one of the two polypeptide chains has sequence identity of at least 60%, at least 65%, at least 70%, at least 75%, at least 80%, at least 85%, at least 90%, at least 91%, at least 92%, at least 93%, at least 94%, or at least 95%, compared to the A chain or B chain of native insulin, but is not particularly limited thereto, and an ordinary person skilled in the art can easily understand by comparing the sequence consisting of two polypeptide chains with that of the A chain or B chain of native insulin.

Native insulin is a hormone secreted by the pancreas to generally promote glucose uptake and inhibit fat breakdown, and thus functions to control blood glucose levels. Insulin is generated from the processing of its precursor, proinsulin, which does not have the function of controlling blood glucose levels. Insulin consists of two polypeptide chains, that is, the A chain and B chain which have 21 and 30 amino acids, respectively, and they are interlinked by two disulfide bridges. The A chain and B chain of native insulin include amino acid sequences represented by SEQ ID NOS: 1 and 2 below, respectively.
A chain: 
Gly-Ile-Val-Glu-Gln-Cys-Cys-Thr-Ser-Ile-Cys-Ser-Leu-Tyr-Gln-Leu-Glu-Asn-Tyr-
Cys-Asn (SEQ ID NO: 1)
B chain:

Phe-Val-Asn-Gln-His-Leu-Cys-Gly-Ser-His-Leu-Val-Glu-Ala-Leu-Tyr-Leu-Val-Cys

-Gly-Glu-Arg-Gly-Phe-Phe-Tyr-Thr-Pro-Lys-Thr (SEQ ID NO: 2)

According to an exemplary embodiment of the present invention, the insulin analogue described in the present invention may be those which have a reduced binding affinity for receptors while having a blood glucose level-controlling function *in vivo* as in native insulin. More specifically, the insulin analogue may have a blood glucose level-lowering ability *in vivo.*

The type and size of the insulin analogue are not particularly limited as long as the insulin analogue can exhibit low receptor-mediated internalization and/or receptor-mediated clearance. Accordingly, the insulin analogue of the present invention can exhibit a significantly increased blood half-life compared to native insulin.

The insulin analogue of the present invention includes inverted insulin, derivatives of native insulin, fragments of native insulin, *etc.* The insulin analogue may be prepared by the solid phase method as well as genetic recombination methods, but the method of preparing the insulin analogue is not limited thereto. Meanwhile, the insulin analogue of the present invention not only includes those insulin analogues prepared by genetic recombination methods but also includes all of the insulin analogues with a reduced binding affinity for an insulin receptor.

As used herein, the term "derivative of native insulin" refers to peptides which have at least one difference in the amino acid sequence compared to native insulin; modified peptides prepared by modification of the sequence of native insulin; and mimetics of native insulin capable of regulating *in vivo* blood glucose levels as in native insulin. These derivatives of native insulin may be those which have the function of regulating *in vivo* blood glucose levels.

Specifically, the derivatives of native insulin may be prepared using any one method selected from substitution, addition, deletion, modification, and a combination thereof with respect to a part of the amino acids of native insulin.

Specifically, the derivatives of native insulin may be those which show a sequence homology of at least 80% in the amino acid sequence compared to each of the A chain and the B chain of native insulin, and/or are in the form where some groups of an amino acid residue of insulin are altered by chemical substitution (*e.g.*, *alpha*-methylation, *alpha*-hydroxylation), deletion (*e.g.,* deamination), or modification (*e.g., N*-methylation), but the sequence homology and the forms of the derivatives of native insulin are not limited thereto.

The derivatives of native insulin that can be applied to the present invention may be prepared by a combination of various methods used for the preparation of derivatives.

Additionally, the modification for the preparation of the derivatives of native insulin includes all of a modification using an L-type or D-type amino acid, and/or a non-native amino acid; and/or alteration or post-translational modification of a native sequence (*e.g.*, methylation, acylation, ubiquitination, intramolecular covalent bonding, *etc*.)*.*

Additionally, the derivatives of native insulin also include those in which one or more amino acids are added to the amino- and/or carboxy-terminus of native insulin.

As the amino acids to be substituted or added, atypical or non-naturally occurring amino acids as well as the 20 amino acids conventionally observed in human proteins may be used. Commercial sources of atypical amino acids include Sigma-Aldrich, ChemPep Inc., Genzyme Pharmaceuticals, *etc.* The peptide sequences including these amino acids and atypical peptide sequences may be synthesized by or purchased from commercial suppliers, *e.g.,* American Peptide Company, Bachem (USA), or Anygen (Korea), but the methods of obtaining these peptide sequences are not particularly limited thereto.

As used herein, the term "fragment of native insulin or fragment of a derivative of native insulin" refers to a form of native insulin or a derivative of native insulin in which one or more amino acids are deleted from the amino- or carboxy-terminus of native insulin or a derivative of native insulin. Such fragment of native insulin or a derivative of native insulin can have the function of regulating blood glucose levels *in vivo.*

Additionally, the insulin analogue of the present invention may include those which are prepared using each of the methods used for the preparation of the derivatives and fragments of native insulin described above independently or prepared using a combined method thereof.

Specifically, the insulin analogue according to the present invention may include a modification in a particular amino acid residue in the A chain and B chain of native insulin, and specifically, these insulin analogues may be those in which particular amino acid residues of the A chain of native insulin are modified and/or particular amino acid residues of the B chain of native insulin are modified.

Specifically, the insulin analogue according to the present invention may be an insulin analogue which has a reduced binding affinity for an insulin receptor compared to native insulin and includes modification and/or deletion in at least one amino acid of the A chain or the B chain of native insulin. For example, the insulin analogue may be one in which a part of the amino acids in native insulin is modified in the form of addition, deletion, substitution, and a combination thereof and thereby its binding affinity for an insulin receptor is reduced compared to native insulin.

Specifically, the insulin analogue may be an insulin analogue in which at least one amino acid, selected from the group consisting of the 1^{st} amino acid, the 2^{nd} amino acid, the 3^{rd} amino acid, the 5^{th} amino acid, the 8^{th} amino acid, the 10^{th} amino acid, the 12^{th} amino acid, the 16^{th} amino acid, the 23^{rd} amino acid, the 24^{th} amino acid, the 25^{th} amino acid, the 26^{th} amino acid, the 27^{th} amino acid, the 28^{th} amino acid, the 29^{th} amino acid, and the 30^{th} amino acid of the insulin B chain, and the 1^{st} amino acid, the 2^{nd} amino acid, the 5^{th} amino acid, the 8^{th} amino acid, the 10^{th} amino acid, the 12^{th} amino acid, the 14^{th} amino acid, the 16^{th} amino acid, the 17^{th} amino acid, the 18^{th} amino acid, the 19^{th} amino acid, and the 21^{st} amino acid of the insulin A chain, is substituted with a different amino acid; or more specifically, an insulin analogue in which at least one amino acid, selected from the group consisting of the 8^{th} amino acid, the 23^{rd} amino acid, the 24^{th} amino acid, and the 25^{th} amino acid of the insulin B chain, and the 1^{st} amino acid, the 2^{nd} amino acid, the 14^{th} amino acid, and the 19^{th} amino acid of the insulin A chain is substituted with a different amino acid.

Specifically, the insulin analogue may be one in which at least 1 amino acid, at least 2 amino acids, at least 3 amino acids, at least 4 amino acids, at least 5 amino acids, at least 6 amino acids, at least 7 amino acids, at least 8 amino acids, at least 9 amino acids, at least 10 amino acids, at least 11 amino acids, at least 12 amino acids, at least 13 amino acids, at least 14 amino acids, at least 15 amino acids, at least 16 amino acids, at least 17 amino acids, at least 18 amino acids, at least 19 amino acids, at least 20 amino acids, at least 21 amino acids, at least 22 amino acids, at least 23 amino acids, at least 24 amino acids, at least 25 amino acids, at least 26 amino acids, or at least 27 amino acids in the amino acids described above, are substituted with a different amino acid, but the insulin analogue is not limited thereto.

The amino acid residues in the positions described above may be substituted with alanine, glutamic acid, asparagine, isoleucine, valine, glutamine, glycine, lysine, histidine, cysteine, phenylalanine, tryptophan, proline, serine, threonine, and/or aspartic acid. Additionally, those insulin analogues which have a reduced binding affinity for an insulin receptor due to deletion in at least one amino acid in the A chain or the B chain of native insulin can belong to the scope of the present invention, but any insulin analogue with a reduced binding affinity for an insulin receptor can be included without limitation.

More specifically, the insulin analogue may be one which includes the A chain of SEQ ID NO: 3 represented by General Formula 1 below and the B chain of SEQ ID NO: 4 represented by General Formula 2 below. Additionally, the insulin analogue may be in a form where the sequences of the A chain and the B chain are interlinked by a disulfide bond, but the form of the insulin analogue is not limited thereto.
[General Formula 1] 
Xaa1-Xaa2-Val-Glu-Gln-Cys-Cys-Thr-Ser-Ile-Cys-Ser-Leu-Xaa14-Gln-Leu-Glu-As
n-Xaa19-Cys-Asn (SEQ ID NO: 3)
 In General Formula 1 above,
Xaal is glycine or alanine,
Xaa2 is isoleucine or alanine,
Xaa14 is tyrosine, glutamic acid, or asparagine, and
Xaa19 is tyrosine or alanine.
[General Formula 2] 
Phe-Val-Asn-Gln-His-Leu-Cys-Xaa8-Ser-His-Leu-Val-Glu-Ala-Leu-Tyr-Leu-Val-C
ys-Gly-Glu-Arg-Xaa23-Xaa24-Xaa25-Tyr-Thr-Pro-Lys-Thr (SEQ ID NO: 4)
 In General Formula 2 above,
Xaa8 is glycine or alanine,
Xaa23 is glycine or alanine,
Xaa24 is phenylalanine or alanine, and
Xaa25 is phenylalanine or alanine.

More specifically, the insulin analogue may include:
(i) the A chain of General Formula 1 above in which Xaa1 is alanine, Xaa2 is isoleucine, Xaa14 is tyrosine, and Xaa19 is tyrosine, and the B chain of General Formula 2 in which Xaa8 is glycine, Xaa23 is glycine, Xaa24 is phenylalanine, and Xaa25 is phenylalanine;
(ii) the A chain of General Formula 1 in which Xaa1 is glycine, Xaa2 is alanine, Xaa14 is tyrosine, and Xaa19 is tyrosine, and the B chain of General Formula 2 in which Xaa8 is glycine, Xaa23 is glycine, Xaa24 is phenylalanine, and Xaa25 is phenylalanine;
(iii) the A chain of General Formula 1 in which Xaa1 is glycine, Xaa2 is isoleucine, Xaa14 is glutamic acid or asparagine, and Xaa19 is tyrosine, and the B chain of General Formula 2 in which Xaa8 is glycine, Xaa23 is glycine, Xaa24 is phenylalanine, and Xaa25 is phenylalanine;
(iv) the A chain of General Formula 1 in which Xaa1 is glycine, Xaa2 is isoleucine, Xaa14 is tyrosine, Xaa19 is alanine in General Formula 1 above, and the B chain of General Formula 2 in which Xaa8 is glycine, Xaa23 is glycine, Xaa24 is phenylalanine, and Xaa25 is phenylalanine;
(v) the A chain of General Formula 1 in which Xaa1 is glycine, Xaa2 is isoleucine, Xaa14 is tyrosine, and Xaa19 is tyrosine, and the B chain of General Formula 2 in which Xaa8 is alanine, Xaa23 is glycine, Xaa24 is phenylalanine, and Xaa25 is phenylalanine;
(vi) the A chain of General Formula 1 in which Xaa1 is glycine, Xaa2 is isoleucine, Xaa14 is tyrosine, and Xaa19 is tyrosine, and the B chain of General Formula 2 in which Xaa8 is glycine, Xaa23 is alanine, Xaa24 is phenylalanine, and Xaa25 is phenylalanine;
(vii) the A chain of General Formula 1 in which Xaa1 is glycine, Xaa2 is isoleucine, Xaa14 is tyrosine, and Xaa19 is tyrosine, and the B chain of General Formula 2 in which Xaa8 is glycine, Xaa23 is glycine, Xaa24 is alanine, and Xaa25 is phenylalanine; and
(viii) the A chain of General Formula 1 in which Xaa1 is glycine, Xaa2 is isoleucine, Xaa14 is tyrosine, and Xaa19 is tyrosine, and the B chain of General Formula 2 in which Xaa8 is glycine, Xaa23 is glycine, Xaa24 is phenylalanine, and Xaa25 is alanine.

Additionally, the insulin analogue may be one which includes the A chain of SEQ ID NO: 5 represented by General Formula 3 below and the B chain of SEQ ID NO: 6 represented by General Formula 4 below. Additionally, the insulin analogue may in a form where the sequences of the A chain and the B chain are interlinked by a disulfide bond, but the form of the insulin analogue is not limited thereto.
[General Formula 3] 
Xaa1-Ile-Val-Glu-Xaa5-Cys-Cys-Thr-Ser-Ile-Cys-Xaa12-Leu-Xaa14-Gln-Xaa16-G
lu-Asn-Xaa19-Cys-Xaa21 (SEQ ID NO: 5)
 In General Formula 3 above,
Xaa1 is alanine, glycine, glutamine, histidine, glutamic acid, or asparagine,
Xaa5 is alanine, glutamic acid, glutamine, histidine, or asparagine,
Xaa12 is alanine, serine, glutamine, glutamic acid, histidine, or asparagine,
Xaa14 is alanine, tyrosine, glutamic acid, histidine, lysine, aspartic acid, or asparagine,
Xaa16 is alanine, leucine, tyrosine, histidine, glutamic acid, or asparagine,
Xaa19 is alanine, tyrosine, serine, glutamic acid, histidine, threonine, or asparagine, and
Xaa21 is asparagine, glycine, histidine, or alanine.
[General Formula 4] 
Phe-Val-Asn-Gln-His-Leu-Cys-Gly-Ser-His-Leu-Val-Glu-Ala-Leu-Xaa16-Leu-Val-
Cys-Gly-Glu-Arg-Gly-Phe-Xaa25-Tyr-Xaa27-Xaa28-Lys-Thr (SEQ ID NO: 6)
 In General Formula 4 above,
Xaa16 is tyrosine, glutamic acid, serine, threonine, or aspartic acid, or is absent,
Xaa25 is phenylalanine, aspartic acid, or glutamic acid, or is absent,
Xaa27 is threonine or is absent, and
Xaa28 is proline, glutamic acid, or aspartic acid, or is absent.

More specifically, the insulin analogue may be one, in which:
in General Formula 3 above, Xaa1 is glycine, Xaa5 is glutamine, Xaa12 is serine, Xaa14 is glutamic acid or asparagine, Xaa16 is leucine, Xaa19 is tyrosine, Xaa21 is asparagine,
in General Formula 4 above, Xaa16 is tyrosine, Xaa25 is phenylalanine or is absent, Xaa27 is threonine, Xaa28 is proline, glutamic acid, or aspartic acid, or is absent, but the insulin analogue is not particularly limited thereto.

More specifically, the insulin analogue may be one in which:
(1) in the A chain of insulin of SEQ ID NO: 5, Xaa1 is glycine, Xaa5 is glutamine, Xaa12 is serine, Xaa14 is glutamic acid, Xaa16 is leucine, Xaa19 is tyrosine, and Xaa21 is asparagine; and
   in the B chain of insulin of SEQ ID NO: 6, Xaa16 is tyrosine, Xaa25 is phenylalanine, Xaa27 is threonine, Xaa28 is proline, glutamic acid, or aspartic acid, or is absent;
(2) in the A chain of insulin of SEQ ID NO: 5, Xaa1 is glycine, Xaa5 is glutamine, Xaa12 is serine, Xaa14 is asparagine, Xaa16 is leucine, Xaa19 is tyrosine, and Xaa21 is asparagine; and
   in the B chain of insulin of SEQ ID NO: 6, Xaa16 is tyrosine, Xaa25 is phenylalanine, Xaa27 is threonine, Xaa28 is proline, glutamic acid, or aspartic acid, or is absent;
(3) in the A chain of insulin of SEQ ID NO: 5, Xaa1 is glycine, Xaa5 is glutamine, Xaa12 is serine, Xaa14 is glutamic acid, Xaa16 is leucine, Xaa19 is tyrosine, and Xaa21 is asparagine; and,
   in the B chain of insulin of SEQ ID NO: 6, Xaa16 is tyrosine, Xaa25 is absent, Xaa27 is threonine, Xaa28 is proline, glutamic acid, or aspartic acid, or is absent; or
(4) in the A chain of insulin of SEQ ID NO: 5, Xaa1 is glycine, Xaa5 is glutamine, Xaa12 is serine, Xaa14 is alanine, Xaa16 is leucine, Xaa19 is tyrosine, Xaa21 is asparagine; and,
   in the B chain of insulin of SEQ ID NO: 6, Xaa16 is glutamic acid, Xaa25 is absent, Xaa27 is threonine, Xaa28 is proline, glutamic acid, or aspartic acid, or is absent, but the insulin analogue is not particularly limited thereto.

More specifically, the insulin analogue may be one in which:
(1) in General Formula 3 above, Xaa1 is glycine, Xaa5 is glutamine, Xaa12 is serine, Xaa14 is glutamic acid, Xaa16 is leucine, Xaa19 is tyrosine, and Xaa21 is asparagine; and
   in General Formula 4 above, Xaa16 is tyrosine, Xaa25 is absent, Xaa27 is threonine, and Xaa28 is proline;
(2) in General Formula 3 above, Xaa1 is glycine, Xaa5 is glutamine, Xaa12 is serine, Xaa14 is alanine, Xaa16 is leucine, Xaa19 is tyrosine, and Xaa21 is asparagine; and
   in General Formula 4 above, Xaa16 is glutamic acid, Xaa25 is absent, Xaa27 is threonine, and Xaa28 is proline;
(3) in General Formula 3 above, Xaa1 is glycine, Xaa5 is glutamine, Xaa12 is serine, Xaa14 is histidine, Xaa16 is leucine, Xaa19 is tyrosine, and Xaa21 is asparagine; and
   in General Formula 4 above, Xaa16 is tyrosine, Xaa25 is phenylalanine, Xaa27 is threonine, and Xaa28 is proline;
(4) in General Formula 3 above, Xaa1 is glycine, Xaa5 is glutamine, Xaa12 is serine, Xaa14 is lysine, Xaa16 is leucine, Xaa19 is tyrosine, and Xaa21 is asparagine; and
   in General Formula 4 above, Xaa16 is tyrosine, Xaa25 is phenylalanine, Xaa27 is threonine, and Xaa28 is proline;
(5) in General Formula 3 above, Xaa1 is glycine, Xaa5 is glutamine, Xaa12 is serine, Xaa14 is tyrosine, Xaa16 is leucine, Xaa19 is glutamic acid, and Xaa21 is asparagine; and
   in General Formula 4 above, Xaa16 is tyrosine, Xaa25 is phenylalanine, Xaa27 is threonine, and Xaa28 is proline;
(6) in General Formula 3 above, Xaa1 is glycine, Xaa5 is glutamine, Xaa12 is serine, Xaa14 is tyrosine, Xaa16 is leucine, Xaa19 is serine, and Xaa21 is asparagine; and
   in General Formula 4 above, Xaa16 is tyrosine, Xaa25 is phenylalanine, Xaa27 is threonine, and Xaa28 is proline;
(7) in General Formula 3 above, Xaa1 is glycine, Xaa5 is glutamine, Xaa12 is serine, Xaa14 is tyrosine, Xaa16 is leucine, Xaa19 is threonine, and Xaa21 is asparagine; and
   in General Formula 4 above, Xaa16 is tyrosine, Xaa25 is phenylalanine, Xaa27 is threonine, and Xaa28 is proline;
(8) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is glutamic acid, Xaa25 is phenylalanine, Xaa27 is threonine, and Xaa28 is proline;
(9) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is serine, Xaa25 is phenylalanine, Xaa27 is threonine, and Xaa28 is proline;
(10) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is threonine, Xaa25 is phenylalanine, Xaa27 is threonine, and Xaa28 is proline;
(11) in General Formula 3 above, Xaa1 is glycine, Xaa5 is glutamine, Xaa12 is serine, Xaa14 is alanine, Xaa16 is leucine, Xaa19 is tyrosine, and Xaa21 is asparagine; and
   in General Formula 4 above, Xaa16 is tyrosine, Xaa25 is phenylalanine, Xaa27 is threonine, and Xaa28 is proline;
(12) in General Formula 3 above, Xaa1 is glycine, Xaa5 is glutamine, Xaa12 is serine, Xaa14 is aspartic acid, Xaa16 is leucine, Xaa19 is tyrosine, and Xaa21 is asparagine; and
   in General Formula 4 above, Xaa16 is tyrosine, Xaa25 is phenylalanine, Xaa27 is threonine, and Xaa28 is proline;
(13) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is aspartic acid, Xaa25 is phenylalanine, Xaa27 is threonine, and Xaa28 is proline;
(14) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is tyrosine, Xaa25 is aspartic acid, Xaa27 is threonine, and Xaa28 is proline; or
(15) in General Formula 3 above, Xaa1 is glycine, Xaa5 is glutamine, Xaa12 is serine, Xaa14 is tyrosine, Xaa16 is leucine, Xaa19 is tyrosine, and Xaa21 is asparagine; and
   in General Formula 4 above, Xaa16 is tyrosine, Xaa25 is glutamic acid, Xaa27 is threonine, and Xaa28 is proline, but the insulin analogue is not particularly limited thereto.

However, the insulin analogue is not limited to the above embodiments. For example, those peptides which have a reduced binding affinity for an insulin receptor compared to native insulin, while containing the characteristic amino acid residues described above and having a homology to that of the subject insulin analogue of at least 70%, specifically at least 80%, more specifically at least 90%, and even more specifically at least 95% are also included to the scope of the present invention.

As used herein, the term "homology" refers to the degree of sequence similarity to an amino acid sequence of a native (wild-type) protein or a polynucleotide sequence encoding the same, and it includes those sequences which have the sequence identity of the percentages described above or higher to the amino acid sequence or polynucleotide sequence of the present invention. These homologies can be determined by comparing two sequences with the naked eye, or alternatively, they can be determined using a bioinformatic algorithm that aligns the sequences to be compared and analyzes the degree of homology. The homology between the two amino acid sequences can be expressed as a percentage. Useful automated algorithms are available in the GAP, BESTFIT, FASTA, and TFASTA computer software modules of the Wisconsin Genetics Software Package (Genetics Computer Group, Madison, WI, USA). The automated array algorithms in this module include Needleman & Wunsch, Pearson & Lipman, and Smith & Waterman sequence alignment algorithms. Algorithm and homology determination for other useful arrays are automated in software including FASTP, BLAST, BLAST2, PSIBLAST, and CLUSTAL W.

Polynucleotides encoding the insulin analogue can be isolated or prepared using standard molecular biology techniques. For example, the polynucleotides encoding the insulin analogue can be amplified by polymerase chain reaction (PCR) from the gene sequence of native insulin (NM_000207.2, NCBI) using appropriate primer sequences or may be prepared using standard synthetic techniques using an automated DNA synthesizer. The polynucleotide may be used interchangeably with nucleic acid in the present invention.

The polynucleotide encoding the insulin analogue may include those polynucleotides which encode the amino acid sequences of the A and B chains described above, but the polynucleotide is not limited thereto. For example, those polynucleotides encoding the peptides which have a homology to the above sequences of at least 70%, specifically at least 80%, more specifically at least 90%, and even more specifically at least 95% and have a reduced binding affinity for an insulin receptor compared to native insulin are also included in the scope of the present invention, in addition to the polynucleotide sequences described above.

Meanwhile, a conjugate with respect to the native insulin or an analogue thereof may be one represented by Formula 2 below:

[Formula 2] X-L-F

in which, in Formula 2 above,
X is native insulin or an insulin analogue with a reduced binding affinity for an insulin receptor compared to native insulin,
L, being a linker, is polyethylene glycol having a size of greater than 0 kDa to less than 3.4 kDa, and
F is a material capable increasing *in vivo* half-life of X.

The conjugate represented by Formula 2 above may be one in which receptor-mediated clearance (RMC) is reduced, but the conjugate is not particularly limited thereto.

In the conjugate represented by Formula 2 above, L may be linked to the amino terminal region of the beta chain of native insulin or an analogue thereof (*i.e.,* X), but the conjugate is not particularly limited thereto.

In the present invention, the term "N-terminal region or amino-terminal region" refers to the amino-terminal region of a peptide or protein. For example, the "N-terminal region" can include not only the most terminal amino acid residue of the N-terminal region but also the amino acid residues adjacent to the N-terminal amino acid residue, and specifically, the 1^{st} amino acid residue to the 20^{th} amino acid residue from the most terminus, but the N-terminal region is not particularly limited thereto.

Meanwhile, in the conjugate according to the present invention, X and F may be linked to each other through L by a covalent chemical bond, a non-covalent chemical bond, or a combination thereof, and specifically, X and F may be linked to each other through L by a covalent chemical bond.

In Formula 1 or 2 above, "-" may denote a covalent chemical bond or a non-covalent chemical bond, and more specifically a covalent chemical bond, but is not particularly limited thereto.

The conjugate of Formula 1 or 2 above has a structure in which X, L, and F are linked in this order.

In a specific embodiment, both ends of L are respectively linked to an amine group or thiol group of F *(e.g.,* an immunoglobulin Fc region) and an amine group or thiol group of X to prepare the conjugate. The amine group may be primary amine or secondary amine.

The amine group may be located at the N-terminus or a side chain of a lysine residue of a polypeptide, such as a physiologically active polypeptide or an immunoglobulin Fc region; and the thiol group may be located at a cysteine residue of a polypeptide, such as a physiologically active polypeptide or an immunoglobulin Fc region.

The amine group of a polypeptide, such as a physiologically active polypeptide or an immunoglobulin Fc region, may form a covalent bond by reacting with an aldehyde or *N*-hydroxysuccinimide ester.

The thiol group of a polypeptide, such as a physiologically active polypeptide or an immunoglobulin Fc region, may form a covalent bond by reacting with maleimide, iodoacetamide, vinylsulfone, pyridyl disulfide, or a thiol group.

Specifically, before forming a conjugate, L may include a reactive group which can bind to F and X at both ends thereof, respectively, specifically a reactive group which can bind to an amine group located at the N-terminus or a lysine residue or a thiol group in a cysteine residue of X; or an amine group located at the N-terminus of or a lysine residue or a thiol group in a cysteine residue of F, but the reactive group is not limited thereto.

L, before being linked to both X and F, can have at least two terminal functional groups, specifically two or three terminal functional groups, and more specifically two terminal functional groups.

Specifically, L may be a homofunctional PEG in which the types of all of the at least two functional groups are the same, or a heterofunctional PEG in which the type of at least one functional group differs from that (those) of the other functional group(s). For example, the PEG may be in a form with two ends where one end of the PEG is aldehyde while the other end is maleimide.

Additionally, L may be a homofunctional PEG in which both ends or all of three ends are aldehyde.

For example, L may be a PEG having a propionaldehyde group or butyraldehyde group at both ends, but is not particularly limited thereto.

When L has a functional group of reactive aldehyde at both ends, it is effective for L to be linked to a physiologically active polypeptide and an immunoglobulin Fc region at both ends, respectively, while minimizing the occurrence of non-specific reactions. The final product formed through reductive amination by an aldehyde bond is significantly more stable compared to those by an amide bond. The aldehyde functional group reacts selectively at the N-terminus at low pH and can form a covalent bond with a lysine residue at high pH (*e.g.,* pH 9.0).

The terminal functional group of L described above may be an amine-reactive functional group or a thiol-reactive functional group.

More specifically, the at least one terminal functional group of L may be each independently selected from the group consisting of aldehyde, maleimide, succinimide, vinylsulfone, thiol, C₆₋₂₀ aryl disulfide, C₅₋₂₀ heteroaryl disulfide, and halogenated acetamide, and even more specifically, may be each independently selected from the group consisting of aldehyde, maleimide, succinimide, vinylsulfone, thiol, *ortho*-pyridyl disulfide, and iodoacetamide, but the terminal functional group is not particularly limited thereto.

As the functional groups of L, the derivatives known in the art and the derivatives that can easily be prepared at the technical level of the art as well as those types described above are also included in the scope of the present invention.

The aldehyde group may be an alkyl aldehyde (*e.g.,* C₂₋₆ alkyl aldehyde), and specifically a propionaldehyde group, a butyraldehyde group, *etc.,* but the aldehyde group is not particularly limited thereto.

The succinimide group may be succinimidyl valerate, succinimidyl methylbutanoate, succinimidyl methylpropionate, succinimidyl butanoate, succinimidyl propionate, hydroxy succinimidyl (specifically *N*-hydroxysuccinimidyl), succinimidyl carboxymethyl, or succinimidyl carbonate. The succinimide group may have an appropriate form so as to be linked to a target functional group located at a physiologically active polypeptide or an immunoglobulin Fc region. For example, the succinimide group may be a *N*-hydroxysuccinimidyl ester.

Additionally, when a PEG having a hydroxy functional group at both ends is used, the hydroxy group can be activated into various reactive groups described above by known chemical reactions.

In the case of a peptide linker used in a fusion protein obtained by a conventional in-frame fusion method, the peptide linker can easily be cleaved by protease *in vivo* and thus a sufficient effect of increasing the serum half-life of an active drug by a carrier cannot be obtained as expected. Therefore, in the present invention, a conjugate can be prepared using polyethylene glycol, which is a non-peptide linker. PEG having resistance to protease may be used as a non-peptide linker to maintain the blood half-life of a given peptide similar to a carrier. The molecular weight of polyethylene glycol is in the range of less than 3.4 kDa, but its molecular weight is not limited thereto.

Meanwhile, with respect to the molecular weight of polyethylene glycol used in the present invention, all of those explained previously will apply to those described above and those to be described later.

In a conjugate according to the present invention, "F" refers to a material which can increase *in vivo* half-life of a physiologically active material. In the present invention, the term "F" may be used interchangeably with "biocompatible material" or carrier.

In the present invention, the "biocompatible material or a material capable of increasing *in vivo* half-life" is one moiety that constitutes the conjugate.

The type of the biocompatible material or carrier is not limited as long as it is a material which is linked to a target physiologically active material and is thereby capable of extending *in vivo* half-life of the same. As non-limiting examples, the biocompatible material or carrier may be selected from the group consisting of polymers, fatty acids, cholesterol, albumin and a fragment thereof, albumin-binding materials, a polymer of repeating units of a particular amino acid sequence, antibodies, antibody fragments, FcRn-binding materials, *in vivo* connective tissues, nucleotides, fibronectin, transferrin, saccharides, heparin, and elastin.

The polymer may be selected from the group consisting of polyethylene glycol, polypropylene glycol, an ethylene glycol-propylene glycol copolymer, polyoxyethylated polyol, polyvinyl alcohol, a polysaccharide, dextran, polyvinyl ethyl ether, a biodegradable polymer, a lipid polymer, chitins, hyaluronic acid, an oligonucleotide, and a combination thereof, but the polymer is not particularly limited thereto.

The biocompatible material or carrier may be covalently or non-covalently linked to X. Additionally, the FcRn-binding material may be a polypeptide including an immunoglobulin Fc region, and specifically an immunoglobulin Fc region (*e.g.,* an IgG Fc).

When albumin is used as a carrier, technologies which can directly covalently link albumin or a fragment thereof to a physiologically active material through a linker, thereby increasing *in vivo* stability of the physiologically active material, can be used. Additionally, technologies which, although not being capable of directly linking albumin to a physiologically active material, can link an albumin-binding material such as an albumin-specific binding antibody or an antibody fragment thereof to a physiologically active material to thereby link the physiologically active material to albumin and technologies which can link a particular peptide/protein having a binding affinity for albumin (*e.g*., the albumin-binding peptide produced using the Albumod technology of Affibody Company) to a physiologically active material may be used, and technologies which can link fatty acids, *etc.* having a binding affinity for albumin may be used, but the technologies are not limited thereto, but any technology, linking methods, *etc.* that can increase *in vivo* stability of a physiologically active material using albumin can be included.

To increase the *in vivo* half-life of a physiologically active material, technologies which can link an antibody or an antibody fragment, as a carrier, to a physiologically active material may also be included. The antibody or antibody fragment may be an antibody or antibody fragment having an FcRn-binding region, and it may be any antibody fragment which does not include an FcRn-binding region such as Fab, *etc.* The CovX-body technology by CovX company using catalytic antibody may be included, and technologies that can increase *in vivo* half-life of a physiologically active material using an immunoglobulin Fc region may be included in the present invention.

Additionally, technologies which can link a fragment of a peptide or protein, as a carrier, to a physiologically active material so as to increase *in vivo* half-life may also be included in the present invention. The fragment of a peptide or protein to be used may be an elastin-like polypeptide (ELP) consisting of a polymer of repeating units of a combination of particular amino acid sequences, and the Xten technology employing an artificial polypeptide PEG by Versartis Inc. Additionally, the structure inducing probe (SIP) technology by Zealand company that can increase *in vivo* half-life of a physiologically active material using multi-lysine and the CTP fusion technology by Prolor Biotech Inc. are also included in the present invention, and transferrin which is known to have high *in vivo* stability, or fibronectin (a constituting component of connective tissue) or a derivative thereof, *etc.* may also be included. The peptide or protein to be linked to a physiologically active material is not limited to those described above, but any peptide or protein that can increase the *in vivo* half-life of a physiologically active material is included in the scope of the present invention.

Additionally, the carrier to be used so as to increase the *in vivo* half-life may be a non-peptide material such as a polysaccharide or fatty acids, *etc.*

When an immunoglobulin is used, the linker for linking an Fc region to a physiologically active material and the method of linking may be a non-peptide linkage using polyethylene glycol. The Fc region and the physiologically active material may be linked at a ratio of 1:1 or 1:2, but the ratio is not limited thereto. Specifically, the Fc region may be in the form of a dimer, and in a form where one molecule of a physiologically active material may be linked to a single chain of an immunoglobulin Fc region in a dimeric form, or in a form where one molecule of a physiologically active material may be linked to each of two chains of an immunoglobulin Fc region in a dimeric form, respectively, but the linkage form is not particularly limited thereto.

Additionally, in the conjugate, with respect to Formula 1 or Formula 2 above, when F is an immunoglobulin Fc region, L may be linked to the N-terminal region of F, but is not particularly limited thereto.

Since an immunoglobulin Fc region is a biodegradable polypeptide that can be metabolized *in vivo,* it is safe for use as a drug carrier. Additionally, the immunoglobulin Fc region has a relatively low molecular weight compared to the whole molecule of immunoglobulin, it is advantageous in terms of preparation, purification, and yield of a conjugate. In addition, as the Fab region, which exhibits high heterogeneity due to the difference in amino acid sequences from antibody to antibody, is removed, it is expected that the homogeneity of materials can be greatly increased and the risk of inducing blood antigenicity can also be lowered.

As used herein, the term "immunoglobulin Fc region" refers to a protein that includes the heavy-chain constant region 2 (CH2) and heavy-chain constant region 3 (CH3) of an immunoglobulin, excluding the heavy-chain and light-chain variable regions of the immunoglobulin.

The immunoglobulin Fc region may include a hinge region in the heavy-chain constant regions. Additionally, the immunoglobulin Fc region of the present invention may be an extended Fc region which includes a part or the entirety of the heavy chain constant region 1 (CH1) and/or the light chain constant region 1 (CL1), excluding the heavy and light chain variable regions of the immunoglobulin, as long as the immunoglobulin Fc region has an effect substantially the same as or more improved compared to that of native Fc. Additionally, the immunoglobulin Fc region of the present invention may be a region in which a significantly long partial amino acid sequence corresponding to the CH2 and/or CH3 is removed.

Specifically, the immunoglobulin Fc region of the present invention may be 1) a CH1 domain, a CH2 domain, a CH3 domain, and a CH4 domain, 2) a CH1 domain and a CH2 domain, 3) a CH1 domain and a CH3 domain, 4) a CH2 domain and a CH3 domain, and 5) a combination between one or two or more domains selected from a CH1 domain, a CH2 domain, a CH3 domain, and a CH4 domain and a hinge region (or part of a hinge region) of an immunoglobulin (*e.g.*, a combination between a CH2 domain and a CH3 domain and a hinge region or part of the hinge region; and a dimer of two polypeptides with the combination described above), and 6) a dimer between each domain of the heavy chain constant region and the light chain constant region.

Additionally, the immunoglobulin Fc region not only includes its native amino acid sequence but also a sequence variant (mutant) thereof. As used herein, the term "amino acid sequence mutant" refers to a sequence that is different from the native amino acid sequence due to the deletion, insertion, non-conservative or conservative substitution, or a combination thereof of one or more amino acid residues of the native amino acid sequence. For example, in the case of an IgG Fc, amino acid residues at positions 214 to 238, 297 to 299, 318 to 322, or 327 to 331, which are known to be important in binding, may be used as suitable sites for modification.

Additionally, other various kinds of mutants are possible, including one that has a deletion of a region capable of forming a disulfide bond, or a deletion of some amino acid residues at the N-terminus of native Fc or an addition of a methionine residue at the N-terminus of native Fc, *etc.* Further, to remove effector functions, a deletion may occur in a complement-binding site, such as a C1q-binding site and an antibody-dependent cell-mediated cytotoxicity (ADCC) site. Techniques for preparing such sequence derivatives of the immunoglobulin Fc region are disclosed in International Patent Publication Nos. WO 97/34631, WO 96/32478, *etc.*

Amino acid exchanges in proteins and peptides, which do not generally alter the activity of the proteins or peptides, are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly.

In some cases, the Fc region may be modified by phosphorylation, sulfation, acrylation, glycosylation, methylation, farnesylation, acetylation, amidation, *etc.*

The above-described Fc mutants may be those which show biological activity identical to that of the Fc region of the present invention but have improved structural stability against heat, pH, *etc.*

Additionally, the Fc region may be obtained from native forms isolated *in vivo* from humans or animals such as cows, goats, pigs, mice, rabbits, hamsters, rats, guinea pigs, *etc.,* or may be recombinants or derivatives thereof, obtained from transformed animal cells or microorganisms. Herein, the Fc region may be obtained from a native immunoglobulin by isolating a whole immunoglobulin from a living human or animal body and treating the isolated immunoglobulin with protease. When the whole immunoglobulin is treated with papain, it is cleaved into Fab and Fc regions, whereas when the whole immunoglobulin is treated with pepsin, it is cleaved into pF'c and F(ab)₂ fragments. These fragments can be isolated using size exclusion chromatography, *etc.*

In a more specific embodiment, the Fc region may be a recombinant immunoglobulin Fc region obtained from a microorganism with regard to a human-derived Fc region.

Additionally, the immunoglobulin Fc region may be in the form of native glycan, increased or decreased glycans compared to its native type, or in a deglycosylated form. The increase, decrease, or removal of the immunoglobulin Fc glycans may be achieved by conventional methods such as a chemical method, enzymatic method, and genetic engineering method using a microorganism. The immunoglobulin Fc region obtained by removal of glycans from the Fc region shows a significant decrease in binding affinity to the complement (C1q part) and a decrease or loss in antibody-dependent cytotoxicity or complement-dependent cytotoxicity, and thus it does not induce unnecessary immune responses *in vivo.* In this regard, an immunoglobulin Fc region in a deglycosylated or aglycosylated immunoglobulin Fc region may be a more suitable form to meet the original object of the present invention as a drug carrier.

As used herein, the term "deglycosylation" refers to enzymatically removing sugar moieties from an Fc region, and the term "aglycosylation" refers to an unglycosylated Fc region produced in prokaryotes, more specifically, *E. coli.*

Meanwhile, the immunoglobulin Fc region may be derived from humans or animals including cows, goats, pigs, mice, rabbits, hamsters, rats, and guinea pigs, and preferably, it is derived from humans. Additionally, the immunoglobulin (Ig) Fc region may be an Fc region derived from IgG, IgA, IgD, IgE, IgM, or a combination or hybrid thereof. Preferably, it may be derived from IgG or IgM, which are among the most abundant proteins in human blood, and most preferably, it may be derived from IgG, which is known to enhance the half-lives of ligand-binding proteins.

Meanwhile, as used herein, the term "combination" means that polypeptides encoding single-chain immunoglobulin Fc regions of the same origin are linked to a single-chain polypeptide of a different origin to form a dimer or multimer. That is, a dimer or multimer can be prepared from two or more fragments selected from the group consisting of IgG Fc, IgA Fc, IgM Fc, IgD Fc, and IgE Fc fragments.

As used herein, the term "hybrid" means that sequences corresponding to two or more immunoglobulin Fc fragments of different origins are present in a single-chain of an immunoglobulin Fc region. In the present invention, various hybrid forms are possible. That is, the hybrid domain may be composed of one to four domains selected from the group consisting of CH1, CH2, CH3, and CH4 of IgG Fc, IgM Fc, IgA Fc, IgE Fc, and IgD Fc, and may include a hinge region.

Meanwhile, IgG may also be divided into the IgG1, IgG2, IgG3, and IgG4 subclasses, and the present invention may include combinations or hybrids thereof, preferably, the IgG2 and IgG4 subclasses, and most preferably, the Fc region of IgG4 rarely having an effector function such as complement dependent cytotoxicity (CDC). That is, the immunoglobulin Fc region of the present invention to be used as a drug carrier may be an aglycosylated Fc region derived from human IgG4. The Fc region derived from human IgG4 is preferred to non-human derived Fc regions, which can cause undesirable immune responses, such as acting as antigens in the human body thereby producing new antibodies against the antigen, *etc.*

In a more specific embodiment, the physiologically active material (e.g., native insulin or an insulin analogue) and a biocompatible material are linked through polyethylene glycol, which is a linker having a size of greater than 0 kDa to less than 3.4 kDa and is interposed between them, and the biocompatible material may be an FcRn-binding material. The FcRn-binding material may be, for example, an immunoglobulin Fc region, and specifically an IgG Fc region.

Still another aspect of the present invention provides a method for preparing the conjugate.

The conjugate and the components constituting the conjugate are the same as explained above.

In a specific embodiment, the present invention provides a method for preparing the conjugate, which includes:
(a) reacting polyethylene glycol, which has a size of greater than 0 kDa to less than 3.4 kDa and at least two terminal functional groups, with any one of a physiologically active material or a material capable of increasing *in vivo* half-life of the physiologically active material to prepare polyethylene glycol, to which one of the physiologically active material or the material capable of increasing *in vivo* half-life of the physiologically active material is covalently linked and which has at least one terminal functional group; and
(b) reacting the polyethylene glycol, to which one of the physiologically active material or the material capable of increasing *in vivo* half-life of the physiologically active material is covalently linked and which has at least one terminal functional group, prepared in step (a) with the other of a physiologically active material or a material capable of increasing *in vivo* half-life of the physiologically active material to prepare a conjugate in which the physiologically active material and a material capable of increasing *in vivo* half-life of the physiologically active material are covalently linked through polyethylene glycol having a size of greater than 0 kDa to less than 3.4 kDa.

Herein, step (a) is named a primary reaction step and step (b) is named a secondary reaction step, respectively.

The physiologically active material and the size of the polyethylene glycol used in the preparation of the conjugate and the constitution with respect to the linking of the conjugate including terminal functional groups are the same as explained above.

In the above preparation method, the physiologically active material has a functional group that reacts with the terminal functional group of polyethylene glycol and thereby forms a covalent bond, and the functional group may be an amine group or thiol group.

Additionally, in the above preparation method, the material which can increase the half-life of the physiologically active material has a functional group that reacts with the terminal functional group and thereby forms a covalent bond, and the functional group may be an amine group or thiol group.

In the above preparation method, the terminal functional group of the polyethylene glycol may be an amine-reactive functional group or thiol-reactive functional group.

In the above preparation method, the terminal functional group of polyethylene glycol may be selected from the group consisting of aldehyde, maleimide, succinimide, vinylsulfone, thiol, C₆₋₂₀ aryl disulfide, C₅₋₂₀ heteroaryl disulfide, and halogenated acetamide. More specifically, the terminal functional group of polyethylene glycol may be selected from the group consisting of aldehyde, maleimide, succinimide, vinylsulfone, thiol, *ortho*-pyridyl disulfide, and iodoacetamide.

The succinimide may be succinimidyl valerate, succinimidyl methylbutanoate, succinimidyl methylpropionate, succinimidyl butanoate, succinimidyl propionate, *N*-hydroxysuccinimidyl, succinimidyl carboxymethyl, or succinimidyl carbonate, but the succinimide is not particularly limited thereto.

In the primary reaction step or secondary reaction step of the present invention, when the terminal functional group of PEG is aldehyde, a covalent bond may be formed between an amine group of X or amine group of F and the aldehyde group of PEG by reductive amination. For example, the '-NH₂' located at the N-terminus of F and the aldehyde group of PEG can react and form a covalent bond.

The reductive amination can be performed in the presence of a reducing agent. The reducing agent may be contained at a final concentration of 1 mM to 20 mM in the primary reaction step and at a final concentration of 1 mM to 100 mM in the secondary reaction step, but the concentrations are not particularly limited thereto.

In the present invention, the reducing agent refers to all of the reducing agents known in the art being capable of forming a covalent bond by reducing a reversible imine double bond, which is formed by linking the aldehyde group *(i.e.,* a functional group of PEG) and the amine group of a polypeptide (a physiologically active polypeptide or immunoglobulin Fc region). For the purpose of the present invention, the reducing agent may be contained in a reaction solution to mediate a covalent bond between PEG and a physiologically active polypeptide or immunoglobulin Fc region.

In the present invention, all of the reducing agents conventionally used in the art may be used as the reducing agent. Specifically, the reducing agent may be sodium cyanoborohydride (SCB), borane pyridine complex, sodium borohydride, borane dimethylamine complex, borane trimethylamine complex, or sodium triacetoxyborohydride, but the reducing agent is not limited thereto. The appropriate reducing agent may be freely selected according to the types of X or F and reaction solvent.

Meanwhile, in the primary reaction step or secondary reaction step of the present invention, when the terminal functional group of PEG is a maleimide group, the thiol group in a cysteine residue of X or the thiol group in a cysteine residue of F (*i.e.,* a sulfhydryl moiety) may react with the maleimide group of PEG to form a covalent bond (a thioether bond).

Still another aspect of the present invention provides a long-acting insulin preparation with improved *in vivo* duration and stability.

The conjugate is the same as explained above.

Meanwhile, as formulations that can increase bioavailability or maintain sustained activity, sustained release formulations by microparticles, nanoparticles, *etc.* using PLGA, hyaluronic acid, chitosan, *etc.* may be contained in the preparation.

Additionally, other types of preparations that can increase bioavailability or maintain sustained activity may include preparations in the form of implant, inhalation, nasal, and patches.

The long-acting preparation may be a long-acting insulin preparation with improved *in vivo* duration and stability compared to that of its native physiologically active material.

When the physiologically active material is native insulin or an analogue thereof, the long-acting preparation may be a pharmaceutical composition for preventing or treating insulin-related diseases (e.g., diabetes), but the physiologically active material is not limited thereto.

As used herein, the term "insulin-related disease" refers to a disease that occurs or progresses due to low or no physiological activity of insulin and may include, for example, diabetes, but the insulin-related disease is not particularly limited thereto.

The pharmaceutical composition containing a conjugate of the present invention may contain a pharmaceutically acceptable carrier. Examples of the pharmaceutically acceptable carrier may include a binder, glidant, disintegrant, excipient, solubilizer, dispersant, stabilizer, suspending agent, coloring agent, fragrance, *etc.* for oral administration; a buffer, preservative, analgesic, solubilizer, isotonic agent, and stabilizer, *etc.* may be mixed to be used for injections; and a base, excipient, lubricant, preservative, *etc.* for topical administration.

The formulation type of the pharmaceutical composition of the present invention may be prepared variously by combining with pharmaceutically acceptable carriers described above. For example, for oral administration, the composition may be formulated into tablets, troches, capsules, elixirs, suspensions, syrups, wafers, *etc.* For injections, the composition may be formulated into unit-dose ampoules or multi-dose forms. The pharmaceutical composition may also be formulated into other forms, such as solutions, suspensions, tablets, pills, capsules, sustained-release preparations, *etc.*

Meanwhile, examples of a suitable carrier, excipient, and diluent may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.*

Additionally, the pharmaceutical composition may further contain a filler, anti-coagulant, lubricant, humectant, fragrance, preservative, *etc.*

Additionally, the conjugate of the present invention may be contained in an amount of 0.001 wt% to 10 wt% relative to the total amount of the composition of the present invention, but the amount is not particularly limited thereto.

Still another aspect of the present invention provides a method for treating insulin-related diseases, which includes administering a conjugate represented by Formula 2 above or a preparation containing the conjugate to a subject in need thereof.

The conjugate according to the present invention is effective for the treatment of diabetes, and thus the treatment of the insulin-related diseases can be promoted by administering a pharmaceutical composition containing the conjugate.

As used herein, the term "administration" refers to introduction of a particular material to a patient by an appropriate manner, and the conjugate may be administered via any of the common routes as long as the drug can arrive at a target tissue via any general route. For example, the administration may be performed intraperitoneally, intravenously, intramuscularly, subcutaneously, intradermally, orally, topically, intranasally, intrapulmonarily, intrarectally, *etc.,* but the administration is not limited thereto. However, since peptides are digested upon oral administration, active ingredients of a composition for oral administration must be coated or formulated for protection against degradation in the stomach. Preferably, the pharmaceutical composition may be administered in an injectable form. Additionally, the pharmaceutical composition may be administered using any apparatus capable of transporting the active ingredients into a target cell.

Additionally, the conjugate or the pharmaceutical composition of the present invention containing the conjugate may be determined by several related factors including the types of diseases to be treated, administration routes, age, sex, and weight of a patient, and severity of the illness, as well as by the types of the drug as an active ingredient. Since the pharmaceutical composition of the present invention has excellent *in vivo* duration and titer, it can considerably reduce the administration frequency and dose of the pharmaceutical preparation of the present invention.

Hereinafter, the present invention will be described in more detail with reference to the following Examples. However, these Examples are for illustrative purposes only and the invention is not limited by these Examples.

### Example 1: Preparation of insulin-3.4 kDa PEG-immunoglobulin Fc conjugate

After dissolving insulin powder (Biocon, India) in 10 mM HCl, for the pegylation of 3.4 K propion-ALD(2) PEG (PEG having a propionaldehyde group, respectively, at both ends, NOF, USA) to the N-terminus of insulin beta chain, insulin (5 mg/mL) and PEG were mixed at a molar ratio of 1:4 and reacted at 4°C for about 2 hours. In particular, the reaction was performed in a mixed solvent of 50 mM sodium citrate buffer (pH 5.0) and 45% isopropanol by adding sodium cyanoborohydride (NaCNBH₃) as a reducing agent. The reaction solution was purified using the SP-HP (GE Healthcare) column, which utilizes a buffer containing sodium citrate (pH 3.0) and 45% EtOH and a KCl concentration gradient.

Then, the purified mono-PEGylated insulin and an immunoglobulin Fc fragment were mixed at a molar ratio of 1:1.2 and the total protein concentration was adjusted to 20 mg/mL, and reacted at 25°C for 15 hours. In particular, the reaction solution was prepared by mixing 100 mM HEPES buffer (pH 8.2) and sodium chloride, and adding 20 mM sodium cyanoborohydride (NaCNBH₃) as a reducing agent thereto.

Upon completion of the reaction, the reaction solution was applied to the Q-HP (GE, USA) column using Tris-HCl (pH 7.5) buffer and a NaCl concentration gradient, applied again to the Source 15ISO (GE, USA) column using ammonium sulfate and a Tris-HCl (pH 7.5) concentration gradient, and thereby the insulin-3.4 kDa PEG-immunoglobulin Fc conjugate was purified.

In the present invention, the insulin-3.4 kDa PEG-immunoglobulin Fc conjugate is used interchangeably with a long-acting insulin conjugate in which a 3.4 kDa PEG linker is bound.

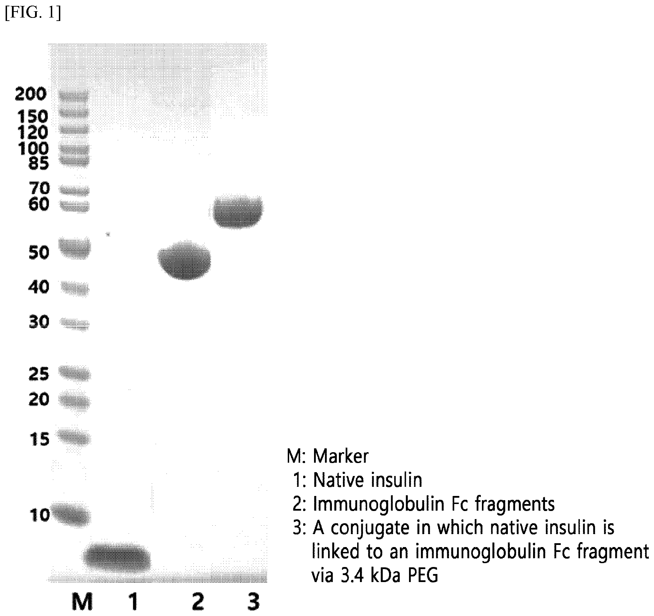

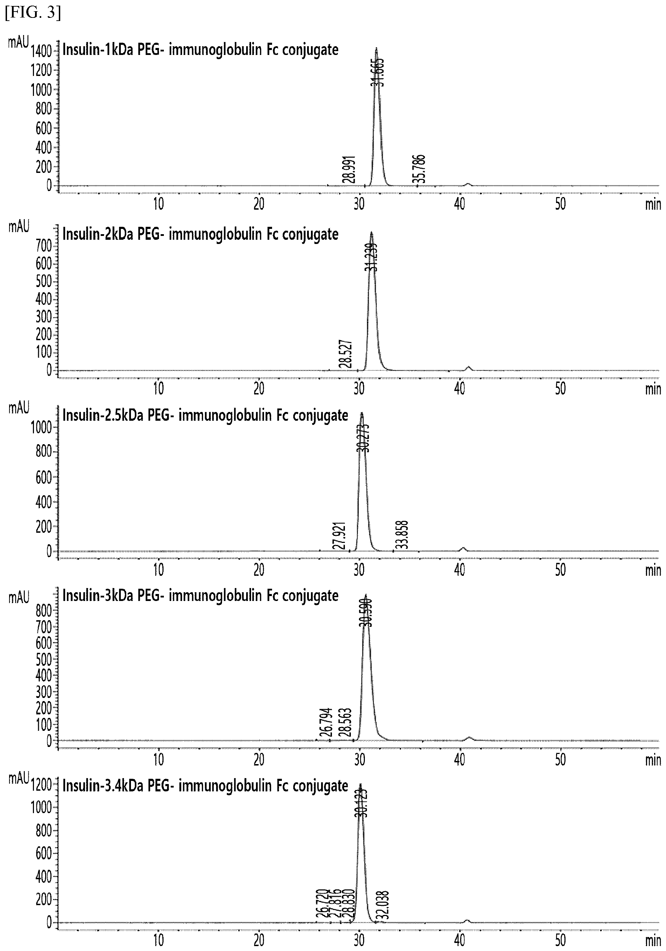

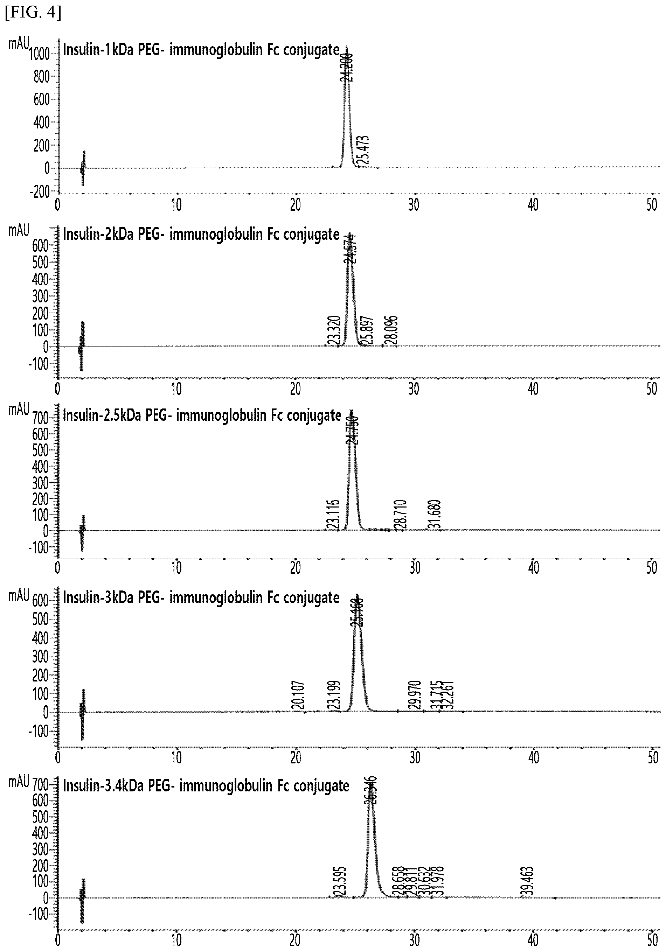

The eluted insulin-3.4 kDa PEG-immunoglobulin Fc conjugate was analyzed by SE-HPLC and RP-HPLC and thereby its purity was confirmed to be 98.5% and 97.4%, respectively (FIGS. 3 and 4). The results of SDS-PAGE purity analysis are shown in FIG. 1.

### Example 2: Preparation of insulin-3.0 kDa PEG-immunoglobulin Fc conjugate

After dissolving insulin powder (Biocon, India) in 10 mM HCl, for the pegylation of 3 K butyr-ALD(2) PEG (PEG having a butyraldehyde group, respectively, at both ends, Hanmi Fine Chemical, Korea) to the N-terminus of insulin beta chain and immunoglobulin Fc, the insulin-3 kDa PEG-immunoglobulin Fc conjugate was prepared and purified according to the conditions of pegylation reaction and purification thereof and the conditions of the reaction with the immunoglobulin Fc fragment and purification thereof in the same manner as in Example 1.

In the present invention, the insulin-3 kDa PEG-immunoglobulin Fc conjugate is used interchangeably with a long-acting insulin conjugate in which a 3 kDa PEG linker is bound.

The eluted insulin-3 kDa PEG-immunoglobulin Fc conjugate was analyzed by SE-HPLC and IE-HPLC and thereby its purity was confirmed to be 99.7% and 97.9%, respectively (FIGS. 3 and 4).

### Example 3: Preparation of insulin-2.5 kDa PEG-immunoglobulin Fc conjugate

After dissolving insulin powder (Biocon, India) in 10 mM HCl, for the pegylation of 2.5 K butyr-ALD(2) PEG (PEG having a butyraldehyde group, respectively, at both ends, Hanmi Fine Chemical, Korea) to the N-terminus of insulin beta chain and immunoglobulin Fc, the insulin-2.5 kDa PEG-immunoglobulin Fc conjugate was prepared and purified according to the conditions of pegylation reaction and purification thereof and the conditions of the reaction with the immunoglobulin Fc fragment and purification thereof in the same manner as in Example 1.

In the present invention, the insulin-2.5 kDa PEG-immunoglobulin Fc conjugate is used interchangeably with a long-acting insulin conjugate in which a 2.5 kDa PEG linker is bound.

The eluted insulin-2.5 kDa PEG-immunoglobulin Fc conjugate was analyzed by SE-HPLC and RP-HPLC and thereby its purity was confirmed to be 99.4% and 99.4%, respectively (FIGS. 3 and 4).

### Example 4: Preparation of insulin-2 kDa PEG-immunoglobulin Fc conjugate

After dissolving insulin powder (Biocon, India) in 10 mM HCl, for the pegylation of 2 K butyr-ALD(2) PEG (PEG having a butyraldehyde group, respectively, at both ends, LAYSAN BIO, USA) to the N-terminus of insulin beta chain and immunoglobulin Fc, the insulin-2 kDa PEG-immunoglobulin Fc conjugate was prepared and purified according to the conditions of pegylation reaction and purification thereof and the conditions of the reaction with the immunoglobulin Fc fragment and purification thereof in the same manner as in Example 1. However, upon completion of the reaction, the reaction solution was applied to the Source 15Q (GE, USA) column using Tris-HCl (pH 7.5) buffer and a NaCl concentration gradient, applied again to the Source 15ISO (GE, USA) column using ammonium sulfate and a Tris-HCl (pH 7.5) concentration gradient, and thereby the insulin-2 kDa PEG-immunoglobulin Fc conjugate was purified.

In the present invention, the insulin-2 kDa PEG-immunoglobulin Fc conjugate is used interchangeably with a long-acting insulin conjugate in which a 2 kDa PEG linker is bound.

The eluted insulin-2 kDa PEG-immunoglobulin Fc conjugate was analyzed by SE-HPLC and RP-HPLC and thereby its purity was confirmed to be 99.9% and 99.4%, respectively (FIGS. 3 and 4).

### Example 5: Preparation of insulin-1 kDa PEG-immunoglobulin Fc conjugate

After dissolving insulin powder (Biocon, India) in 10 mM HCl, for the pegylation of 1 K butyr-ALD(2) PEG (PEG having a butyraldehyde group, respectively, at both ends, NEKTAR, USA) to the N-terminus of insulin beta chain, the insulin-1 kDa PEG-immunoglobulin Fc conjugate was prepared and purified according to the conditions of pegylation reaction and purification thereof and the conditions of the reaction with the immunoglobulin Fc fragment and purification thereof in the same manner as in Example 1.

In the present invention, the insulin-1 kDa PEG-immunoglobulin Fc conjugate is used interchangeably with a long-acting insulin conjugate in which a 1 kDa PEG linker is bound.

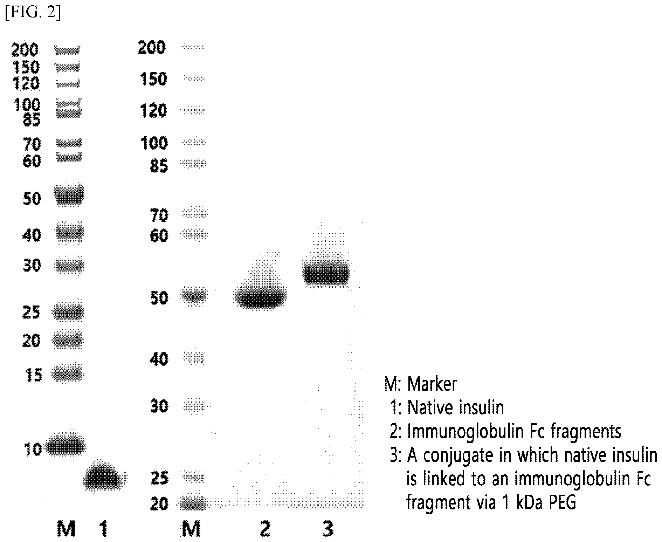

The eluted insulin-1 kDa PEG-immunoglobulin Fc conjugate was analyzed by SE-HPLC and RP-HPLC and thereby its purity was confirmed to be 99.8% and 99.2%, respectively (FIGS. 3 and 4). The results of SDS-PAGE purity analysis are shown in FIG. 2.

### Example 6: Analysis of effect of duration of drug effect of long-acting insulin conjugate in normal rat according to length of PEG linker

The difference in duration of drug effects according to the length of the PEG linker of long-acting insulin conjugates was confirmed in normal rats by administering the long-acting insulin conjugates prepared above, to each of which a 1 kDa, 2 kDa, 2.5 kDa, 3 kDa, or 3.4 kDa PEG linker is bound.

Eight-week-old normal rats were subdivided into a control group (vehicle), a group administered with a long-acting insulin conjugate in which a 1 kDa PEG linker is bound (65.1 nmol/kg), a group administered with a long-acting insulin conjugate in which a 2 kDa PEG linker is bound (65.1 nmol/kg), a group administered with a long-acting insulin conjugate in which a 2.5 kDa PEG linker is bound (65.1 nmol/kg), a group administered with a long-acting insulin conjugate in which a 3 kDa PEG linker is bound (65.1 nmol/kg), and a group administered with a long-acting insulin conjugate in which a 3.4 kDa PEG linker is bound (65.1 nmol/kg), respectively. The normal rats were subcutaneously administered once with the test materials for 3 rats in each group, and their whole blood samples were collected through their caudal veins at 1, 4, 8, 24, 48, and 72 hours, respectively, added into each of 1.5 mL microtubes, centrifuged at 5,000 rpm for 10 minutes at room temperature, and each serum was separated and stored at -20°C, respectively. Each of the concentration of the insulin conjugates contained in the sera for each group was quantified by ELISA assay. The ELISA assay was performed in such a manner that the sera collected each time and anti-human IgG4-HPR (Alpha Diagnosis, #10124) were simultaneously added to each plate coated with insulin monoclonal antibody (ALPCO, #80-INSHU-E10.1), reacted at room temperature for 1 hour, allowed to develop colors with the TMB reagent, and their absorbance was measured at 450 nm, respectively.

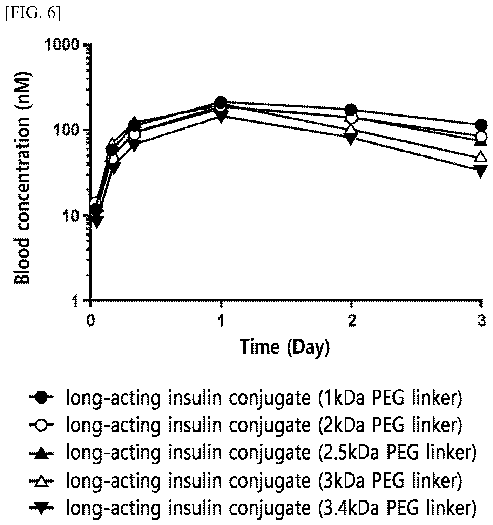

As a result, compared to the group which was administered with the long-acting insulin conjugate in which a 3.4 kDa PEG linker is bound, the group which was administered with the long-acting insulin conjugate in which a 1 kDa PEG linker is bound showed a 1.84-fold increase in AUC; the group which was administered with the long-acting insulin conjugate in which a 2 kDa PEG linker is bound showed a 1.80-fold increase in AUC; the group which was administered with the long-acting insulin conjugate in which a 2.5 kDa PEG linker is bound showed a 1.41-fold increase in AUC; and the group which was administered with the long-acting insulin conjugate in which a 3 kDa PEG linker is bound showed a 1.43-fold increase in AUC, respectively (FIG. 6).

These results suggest that, surprisingly, as the size of the polyethylene glycol used as the linker becomes shorter, the *in vivo* half-life of the insulin conjugate significantly increases and thus can be provided as a stable insulin preparation and effectively used for the treatment of diabetes.

Additionally, as the size of the polyethylene glycol used as the linker becomes shorter, the *in vivo* half-life of a conjugate in which a physiologically active polypeptide is linked can be significantly increased, thus allowing it to be provided as a stable preparation.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A conjugate of Formula 1 below:
[Formula 1] X-L-F
wherein:
X is a physiologically active material;
L, being a linker, is polyethylene glycol having a size of greater than 0 kDa to less than 3.4 kDa; and
F is a material capable increasing *in vivo* half-life of the physiologically active material.

2. The conjugate of claim 1, wherein the conjugate exhibits an increased *in vivo* half-life compared to a conjugate which has the same X and F as the conjugate but has a different L, as a linker, which is polyethylene glycol having a size of 3.4 kDa.

3. The conjugate of claim 1, wherein L is polyethylene glycol having a size of greater than 0 kDa to 3 kDa or less.

4. The conjugate of claim 1, wherein the physiologically active material is selected from the group consisting of toxins; glucagon-like peptide-1 (GLP-1) receptor agonists; glucagon receptor agonists; gastric inhibitory polypeptide (GIP) receptor agonists; fibroblast growth factor (FGF) receptor agonists; cholecystokinin receptor agonists; gastrin receptor agonists; melanocortin receptor agonists; materials binding to two or more receptors among GLP receptor, glucagon receptor, and GIP receptor; somatostatin; peptide YY (PYY); neuropeptide Y (NPY); oxyntomodulin; fibroblast growth factor (FGF); bradykinin; eledoisin; oxytocin; vasopressin; sermorelin; prolactin-releasing peptides; orexin; thyroid-releasing peptides; calmodulin; motilin; vasoactive intestinal peptides; atrial natriuretic peptides (ANP); C-type natriuretic peptides (CNP); neurokinin A; neuromedin; renin; endothelin; sarafotoxin peptides; carsomorphin peptides; dermorphin; dynorphin; endorphin; enkepalin; tumor necrosis factor receptors; urokinase receptors; thymopoietin; thymulin; thymopentin; tymosin; thymic humoral factors; adrenomodullin; allatostatin; amyloid β-protein fragments; antibiotic peptides; antioxidant peptides; bombesin; osteocalcin; CART peptides; E-selectin; intercellular adhesion molecule 1 (ICAM-1); vascular cell adhesion molecule 1 (VCAM-1); leucokine; kringle-5; laminin; inhibin; galanin; fibronectin; pancreastatin; fuzeon; glucagon-like peptides; G protein-coupled receptors; erythropoietic growth factors; leukopoietin; amylin; human growth hormone; growth hormone-releasing hormone; growth hormone-releasing peptides; interferons; interferon receptors; colony-stimulating factors; interleukins; interleukin receptors; enzymes; interleukin-binding proteins; cytokine-binding proteins; macrophage-activating factors; macrophage peptides; B cell factors; T cell factors; protein A; allergy-inhibiting factors; necrosis glycoproteins; immunotoxins; lymphotoxins; tumor necrosis factors; tumor suppressors; transforming growth factors; α-1 antitrypsin; albumin; α-lactalbumin; apolipoprotein-E; erythropoietin; high-glycosylated erythropoietin; angiopoietins; hemoglobins; thrombin; thrombin receptor-activating peptides; thrombomodulin; blood coagulation factor VII; blood coagulation factor VIIa; blood coagulation factor VIII; blood coagulation factor IX; blood coagulation factor XIII; plasminogen activators; fibrin-binding peptides; urokinase; streptokinase; hirudin; protein C; C-reactive protein; renin inhibitors; collagenase inhibitors; superoxide dismutase; leptin; platelet-derived growth factor; epithelial growth factor; epidermal growth factor; angiostatin; angiotensin; bone morphogenetic growth factor; bone morphogenetic protein; calcitonin; insulin; atriopeptin; cartilage-inducing factor; elcatonin; connective tissue-activating factor; tissue factor pathway inhibitor; follicle-stimulating hormone; luteinizing hormone; luteinizing hormone-releasing hormone; nerve growth factors; axogenesis factor-1; brain-natriuretic peptide; glial-derived neurotrophic factor; netrin; neutrophil inhibitory factor; neurotrophic factor; neurturin; parathyroid hormone; relaxin; secretin; somatomedin; insulin-like growth factor; adrenocortical hormone; glucagon; cholecystokinin; pancreatic polypeptides; gastrin-releasing peptides; gastrin inhibitory peptides; corticotropin-releasing factor; thyroid-stimulating hormone; autotaxin; lactoferrin; myostatin; activity-dependent neuroprotective protein (ADNP), β-secretase1 (BACE1), amyloid precursor protein (APP), neural cell adhesion molecule (NCAM), amyloid β, tau, receptor for advanced glycation endproducts (RAGE), α-synuclein, or agonists or antagonists thereof; receptors, receptor agonists; cell surface antigens; monoclonal antibody; polyclonal antibody; antibody fragments; virus-derived vaccine antigens; hybrid polypeptides or chimeric polypeptides that activate at least one receptor agonist; and analogues thereof.

5. The conjugate of claim 4, wherein:
the toxin is selected from the group consisting of maytansine or a derivative thereof, auristatin or a derivative thereof, duocarmycin or a derivative thereof, and pyrrolobenzodiazepine (PBD) or a derivative thereof;
the glucagon-like peptide-1 (GLP-1) receptor agonist is selected from the group consisting of native glucagon-like peptide-1 (GLP-1), native exendin-3, native exendin-4, and analogues thereof;
the FGF receptor agonist is selected from the group consisting of FGF1 or an analogue thereof, FGF19 or an analogue thereof, FGF21 or an analogue thereof, and FGF23 or an analogue thereof;
the interferon is selected from the group consisting of interferon-a, interferon-β, and interferon-γ;
the interferon receptor is selected from the group consisting of interferon-a receptor, interferon-β receptor, interferon-y receptor, and soluble type I interferon receptors;
the interleukin is selected from the group consisting of interleukin-1, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18, interleukin-19, interleukin-20, interleukin-21, interleukin-22, interleukin-23, interleukin-24, interleukin-25, interleukin-26, interleukin-27, interleukin-28, interleukin-29, and interleukin-30;
the interleukin receptor is interleukin-1 receptor or interleukin-4 receptor;
the enzyme is selected from the group consisting of β-glucosidase, α-galactosidase, β-galactosidase, iduronidase, iduronate-2-sulfatase, galactose-6-sulfatase, acid α-glucosidase, acid ceramidase, acid sphingomyelinase, galactocerebrosidase, arylsulfatase A, arylsulfatase B, β-hexosaminidase A, β-hexosaminidase B, heparin *N*-sulfatase, α-D-mannosidase, β-glucuronidase, *N*-acetylgalactosamine-6 sulfatase, lysosomal acid lipase, α-*N*-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, myeloperoxidase, α-galactosidase-A, agalsidase α, agalsidase β, α-L-iduronidase, butyrylcholinesterase, chitinase, glutamate decarboxylase, and imiglucerase;
the interleukin-binding protein is IL-18bp;
the cytokine-binding protein is tumor necrosis factor (TNF)-binding protein;
the nerve growth factors are selected from the group consisting of nerve growth factor, ciliary neurotrophic factor, axogenesis factor-1, brain-natriuretic peptide, glial-derived neurotrophic factor, netrin, neutrophil inhibitory factor, neurotrophic factor, and neurturin;
the myostatin receptor is selected from the group consisting of TNFR (P75), TNFR (P55), IL-1 receptor, VEGF receptor, and B cell activating factor receptor;
the myostatin receptor antagonist is IL1-Ra;
the cell surface antigen is selected from the group consisting of CD2, CD3, CD4, CD5, CD7, CD11a, CD11b, CD18, CD19, CD20, CD23, CD25, CD33, CD38, CD40, CD45, and CD69; and
the antibody fragments are selected from the group consisting of scFv, Fab, Fab', F(ab')₂, and Fd.

6. The conjugate of claim 1, wherein the physiologically active material is native exendin-3 or an analogue thereof; native exendin-4 or an analogue thereof; native insulin or an analogue thereof; native GLP-1 or an analogue thereof; native GLP-2 or an analogue thereof; native oxyntomodulin or an analogue thereof; native glucagon or an analogue thereof; native fibroblast growth factor or an analogue thereof; native ghrelin or an analogue thereof; native calcitonin or an analogue thereof; native granulocyte-colony stimulating factor or an analogue thereof; or a material binding to two or more receptors among GLP receptor, glucagon receptor, and GIP receptor.

7. The conjugate of claim 1, wherein the physiologically active material is native insulin or an insulin analogue which has a reduced binding affinity for an insulin receptor compared to native insulin.

8. The conjugate of claim 7, wherein the insulin analogue has a reduced binding affinity for an insulin receptor compared to native insulin and comprises at least one amino acid modification or deletion in the A chain or B chain of native insulin.

9. The conjugate of claim 8, wherein the insulin analogue is an insulin analogue in which at least one amino acid, selected from the group consisting of the 1^{st} amino acid, the 2^{nd} amino acid, the 3^{rd} amino acid, the 5^{th} amino acid, the 8^{th} amino acid, the 10^{th} amino acid, the 12^{th} amino acid, the 16^{th} amino acid, the 23^{rd} amino acid, the 24^{th} amino acid, the 25^{th} amino acid, the 26^{th} amino acid, the 27^{th} amino acid, the 28^{th} amino acid, the 29^{th} amino acid, and the 30^{th} amino acid of the insulin B chain, and the 1^{st} amino acid, the 2^{nd} amino acid, the 5^{th} amino acid, the 8^{th} amino acid, the 10^{th} amino acid, the 12^{th} amino acid, the 14^{th} amino acid, the 16^{th} amino acid, the 17^{th} amino acid, the 18^{th} amino acid, the 19^{th} amino acid, and the 21^{st} amino acid of the insulin A chain, is substituted with a different amino acid or deleted.

10. The conjugate of claim 9, wherein the insulin analogue is an insulin analogue in which at least one amino acid, selected from the group consisting of the 8^{th} amino acid, the 23^{rd} amino acid, the 24^{th} amino acid, and the 25^{th} amino acid of the native insulin B chain, and the 1^{st} amino acid, the 2^{nd} amino acid, the 14^{th} amino acid, and the 19^{th} amino acid of the native insulin A chain, is substituted with a different amino acid.

11. The conjugate of claim 10, wherein the substituting different amino acid is selected from the group consisting of alanine, glutamic acid, asparagine, isoleucine, valine, glutamine, glycine, lysine, histidine, cysteine, phenylalanine, tryptophan, proline, serine, threonine, and aspartic acid.

12. The conjugate of claim 1, wherein F is selected from the group consisting of polymers, fatty acids, cholesterol, albumin and a fragment thereof, albumin-binding materials, a polymer of repeating units of a particular amino acid sequence, antibodies, antibody fragments, FcRn-binding materials, *in vivo* connective tissues, nucleotides, fibronectin, transferrin, saccharides, heparin, and elastin.

13. The conjugate of claim 12, wherein the polymer is selected from the group consisting of polyethylene glycol, polypropylene glycol, an ethylene glycol-propylene glycol copolymer, polyoxyethylated polyol, polyvinyl alcohol, a polysaccharide, dextran, polyvinyl ethyl ether, a biodegradable polymer, a lipid polymer, chitins, hyaluronic acid, an oligonucleotide, and a combination thereof.

14. The conjugate of claim 1, wherein F is an immunoglobulin Fc region.

15. The conjugate of claim 1, wherein F is an IgG Fc region.

16. A method for preparing the conjugate of claim 1, comprising:
(a) reacting polyethylene glycol, which has a size of greater than 0 kDa to less than 3.4 kDa and at least two terminal functional groups, with any one of a physiologically active material or a material capable of increasing *in vivo* half-life of the physiologically active material to prepare polyethylene glycol, to which one of the physiologically active material or the material capable of increasing *in vivo* half-life of the physiologically active material is covalently linked and which has at least one terminal functional group; and
(b) reacting the polyethylene glycol prepared in step (a) with the other of a physiologically active material or a material capable of increasing *in vivo* half-life of the physiologically active material to prepare a conjugate in which the physiologically active material and a material capable of increasing *in vivo* half-life of the physiologically active material are covalently linked through polyethylene glycol having a size of greater than 0 kDa to less than 3.4 kDa.

17. A long-acting preparation with improved *in vivo* duration and stability comprising the conjugate of any one of claims 1 to 15.

18. A preparation for preventing or treating diabetes comprising the conjugate of any one of claims 7 to 11.

19. A method for treating diabetes comprising comprising administering the preparation for preventing or treating diabetes of claim 18 to a subject in need thereof.


<110>    HANMI PHARM. CO., LTD.

<120>    Long-acting conjugate of a physiologically active material and
         use thereof

<130>    OPA18057-PCT

<150>    KR 10-2017-0015663
<151>    2017-02-03

<150>    KR 10-2017-0127187
<151>    2017-09-29

<160>    6

<170>    KoPatentIn 3.0

<210>    1
<211>    21
<212>    PRT
<213>    Homo sapiens

<400>    1
Gly Ile Val Glu Gln Cys Cys Thr Ser Ile Cys Ser Leu Tyr Gln Leu
  1               5                  10                  15

Glu Asn Tyr Cys Asn
            20


<210>    2
<211>    30
<212>    PRT
<213>    Homo sapiens

<400>    2
Phe Val Asn Gln His Leu Cys Gly Ser His Leu Val Glu Ala Leu Tyr
  1               5                  10                  15

Leu Val Cys Gly Glu Arg Gly Phe Phe Tyr Thr Pro Lys Thr
            20                  25                  30


<210>    3
<211>    21
<212>    PRT
<213>    Artificial Sequence

<220>
<223>    Insulin analog A-chain


<220>
<221>    MISC_FEATURE
<222>    (1)
<223>    Xaa is glycine or alanine


<220>
<221>    MISC_FEATURE
<222>    (2)
<223>    Xaa is isoleucine or alanine

<220>
<221>    MISC_FEATURE
<222>    (14)
<223>    Xaa is tyrosine, glutamic acid, or aspargine


<220>
<221>    MISC_FEATURE
<222>    (19)
<223>    Xaa is tyrosine or alanine


<400>    3
Xaa Xaa Val Glu Gln Cys Cys Thr Ser Ile Cys Ser Leu Xaa Gln Leu
1               5                   10                  15

Glu Asn Xaa Cys Asn
            20


<210>    4
<211>    30
<212>    PRT
<213>    Artificial Sequence

<220>
<223>    Insulin B-chain


<220>
<221>    MISC_FEATURE
<222>    (8)
<223>    Xaa is glycine or alanine


<220>
<221>    MISC_FEATURE
<222>    (23)
<223>    Xaa is glycine or alanine


<220>
<221>    MISC_FEATURE
<222>    (24)
<223>    Xaa is phenylalanine or alanine


<220>
<221>    MISC_FEATURE
<222>    (25)
<223>    Xaa is phenylalanine or alanine


<400>    4
Phe Val Asn Gln His Leu Cys Xaa Ser His Leu Val Glu Ala Leu Tyr
1               5                   10                  15

Leu Val Cys Gly Glu Arg Xaa Xaa Xaa Tyr Thr Pro Lys Thr
            20                  25                  30


<210>    5

<211>    21
<212>    PRT
<213>    Artificial Sequence

<220>
<223>    Insulin analog A-chain


<220>
<221>    MISC_FEATURE
<222>    (1)
<223>    Xaa is alanine, glycine, glutamine, histidine, glutamic acid, or
         asparagine


<220>
<221>    MISC_FEATURE
<222>    (5)
<223>    Xaa is alanine, glutamic acid, glutamine, histidine, or
         asparagine


<220>
<221>    MISC_FEATURE
<222>    (12)
<223>    Xaa is alanine, serine, glutamine, glutamic acid, histidine, or
         asparagine


<220>
<221>    MISC_FEATURE
<222>    (14)
<223>    Xaa is alanine, tyrosine, glutamic acid, histidine, lysine,
         aspartic acid, or asparagine


<220>
<221>    MISC_FEATURE
<222>    (16)
<223>    Xaa is alanine, leucine, tyrosine, histidine, glutamic acid, or
         asparagine


<220>
<221>    MISC_FEATURE
<222>    (19)
<223>    Xaa is alanine, tyrosine, serine, glutamic acid, histidine,
         threonine or asparagine


<220>
<221>    MISC_FEATURE
<222>    (21)
<223>    Xaa is asparagine, glycine, histidine, or alanine


<400>    5
Xaa Ile Val Glu Xaa Cys Cys Thr Ser Ile Cys Xaa Leu Xaa Gln Xaa
1               5                   10                  15

Glu Asn Xaa Cys Xaa
            20

<210>    6
<211>    30
<212>    PRT
<213>    Artificial Sequence

<220>
<223>    Insulin analog B-chain


<220>
<221>    MISC_FEATURE
<222>    (16)
<223>    Xaa is tyrosine, glutamic acid, serine, threonine, or aspartic
         acid, or is absent


<220>
<221>    MISC_FEATURE
<222>    (25)
<223>    Xaa is phenylalanine, aspartic acid, or glutamic acid, or is
         absent


<220>
<221>    MISC_FEATURE
<222>    (27)
<223>    Xaa is threonine, or is absent


<220>
<221>    MISC_FEATURE
<222>    (28)
<223>    Xaa is proline, glutamic acid, or aspartic acid, or is absent


<400>    6
Phe Val Asn Gln His Leu Cys Gly Ser His Leu Val Glu Ala Leu Xaa
 1               5                   10                  15

Leu Val Cys Gly Glu Arg Gly Phe Xaa Tyr Xaa Xaa Lys Thr
            20                  25                  30